(19) Europäisches Patentamt
European Patent Office
Office européen des brevets

(11) EP 4 678 628 A1

(12) # EUROPEAN PATENT APPLICATION

(43) Date of publication:
**14.01.2026 Bulletin 2026/03**

(21) Application number: **24382738.3**

(22) Date of filing: **10.07.2024**

(51) International Patent Classification (IPC):
***C07C 217/48** (2006.01)* ***A61P 25/00** (2006.01)*

(52) Cooperative Patent Classification (CPC):
**C07C 217/48; A61P 25/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Connecta Theapeutics, S.L.**
**08028 Barcelona (ES)**

(72) Inventors:
- **PROUS BLANCAFORT, Josep**
  **E-08028 Barcelona (ES)**
- **PASCUAL GILABERT, Marta**
  **E-08028 Barcelona (ES)**
- **FÀBREGA CASADELLÀ, Jordi**
  **E-08028 Barcelona (ES)**
- **DOMINGO PASCUAL, Irene**
  **E-08028 Barcelona (ES)**

(74) Representative: **ABG Intellectual Property Law, S.L.**
**Avenida de Burgos, 16D**
**Edificio Euromor**
**28036 Madrid (ES)**

(54) **COMPOUNDS AND COMPOSITIONS FOR THE TREATMENT OF NERVOUS SYSTEM DISORDERS**

(57) New 3-phenoxy-3-phenylpropanamine derivatives, compositions thereof and their use as a medicament in the treatment of nervous system disorders associated with cognitive deficits and behavioural dysfunctions, in particular fragile X syndrome and Rett syndrome.

EP 4 678 628 A1

## Description

### FIELD OF THE INVENTION

New 3-phenoxy-3-phenylpropanamine derivatives, compositions thereof and their use as a medicament in the treatment of nervous system disorders associated with cognitive deficits and behavioural dysfunctions, in particular in the treatment of fragile X syndrome and Rett syndrome.

### BACKGROUND OF THE INVENTION

Cognitive deficit is an inclusive term used to describe the impairment of different domains of cognition, wherein cognition is the mental action or process of acquiring knowledge and understanding through thought, experience, and the senses. Cognition encompasses various aspects of high-level intellectual functions and processes such as memory, knowledge, decision making, planning, reasoning, judgment, perception, attention, comprehension, language, and visuospatial function, among others. [Dhakal A, Bobrin BD. Cognitive Deficits. [Updated 2023 Feb 14]. In: StatPearls [Internet]. Treasure Island (FL): StatPearls Publishing; 2024 Jan-. Available from: https://www.ncbi.nlm.nih.gov/books/NBK559052/].

Behavioural dysfunctions, also known as behavioural abnormalities, comprise alterations in emotionality, social interaction and spatial memory, as well as hyperactivity, aggressiveness, stereotypy, and the like, which impact a person's ability to effectively recognize, interpret, control, and express fundamental emotions.

Cognitive deficit and behavioural dysfunctions are not limited to any particular disease or condition but a manifestation of an underlying condition.

Cognitive deficits and behavioural dysfunctions have been observed in a large variety of nervous system disorders. For the purpose of the present invention, nervous system disorders associated with cognitive deficits and behavioural dysfunctions consist of developmental anomalies, such as fragile X syndrome, Rett syndrome, Down syndrome, DiGeorge syndrome (22q11.2 deletion syndrome), Prader-Willi syndrome, and Angelman syndrome; mental, behavioural and neurodevelopmental disorders such as autism spectrum disorder, Asperger syndrome, attention deficit hyperactivity disorder, anxiety, depression, and schizophrenia; and diseases of the nervous system, such as Alzheimer's disease, Huntington's disease, and Parkinson's disease; as defined by the World Health Organization in the International Classification of Diseases 11th Revision (ICD-11, https://icd.who.int/).

Fragile X syndrome (FXS) is the most commonly inherited form of single-gene mutation that causes a range of nervous system manifestations with an estimated frequency of 1:4,000-5,000 worldwide. FXS is typically characterized by mild to severe cognitive deficits with associated mood, social and behavioural dysfunctions including autism spectrum disorder, attention-deficit hyperactivity disorder and aggression [Dionne O, Corbin F. (2021) An "omic" overview of fragile X syndrome. Biology (Basel), 10(5):433]. FXS is caused by a mutation of the fragile X messenger ribonucleoprotein 1 *(FMR1)* gene, which arises from the hypermethylation of a cytosine-guanine-guanine trinucleotide repeat expansion [Protic DD, Aishworiya R, Salcedo-Arellano MJ, et al. Fragile X syndrome: From molecular aspect to clinical treatment. (2022). Int J Mol Sci, 23(4):1935]. A full mutation consists of >200 repeats, resulting in epigenetic silencing of FMR1 leading to loss of expression [Dionne O, Corbin F. An "omic" overview of fragile X syndrome. (2021). Biology (Basel).10(5):433]. Fragile X messenger ribonucleoprotein (FMRP), the product of the *FMR1* gene, is an RNA-binding protein critical to neuronal development, synaptic plasticity, and dendritic spine architecture [Bagni C, Zukin RS. (2019). A Synaptic Perspective of Fragile X Syndrome and Autism Spectrum Disorders. Neuron, Mar 20;101(6):1070-1088].

Rett syndrome (RS) is a severe neurological disorder predominantly affecting females, caused by mutations in the *MECP2* gene. This gene encodes for the MeCP2 protein, a critical transcriptional regulator involved in gene expression, chromatin remodelling, and RNA processing [Lopes, A. G., Loganathan, S. K., & Caliaperumal, J. (2024). Rett Syndrome and the Role of MECP2: Signaling to Clinical Trials. Brain Sciences, 14(2), 120; and Gomes, A. R., Fernandes, T. G., Cabral, J. M., & Diogo, M. M. (2021). Modeling Rett syndrome with human pluripotent stem cells: Mechanistic outcomes and future clinical perspectives. International journal of molecular sciences, 22(7), 3751]. The dysfunction of MeCP2 results in a variety of symptoms including microcephaly, seizures, and intellectual disabilities.

Down syndrome (DS) is a genetic disorder caused by the trisomy of chromosome 21 [Dierssen M. (2012). Down syndrome: the brain in trisomic mode. Nat Rev Neurosci, Dec;13(12):844-58]. DS is the most common genetic form of intellectual disability. It is usually associated with developmental delays, distinct facial features and congenital heart defects [Bull, M. J. (2020). Down syndrome. New England Journal of Medicine, 382(24), 2344-2352].

DiGeorge syndrome, also known as 22q11.2 deletion syndrome (22q11.2DS), is a genetic disorder that arises from a hemizygous deletion on the long arm of chromosome 22q11.2. 22q11.2DS presents a varied combination of anatomical, behavioral, and cognitive dysfunctions, such as cardiac defects, palatal deformities, and facial abnormalities, an increased risk for an array of psychiatric disorders, most notably schizophrenia, though autism spectrum disorder, attention deficit hyperactivity disorder, and anxiety disorders [Smerconish S, Schmitt JE. (2024). Neuroanatomical

Correlates of Cognitive Dysfunction in 22q11.2 Deletion Syndrome. Genes (Basel), 15(4):440].

Prader-Willi syndrome (PWS) is a multisystemic complex genetic disorder caused by lack of expression of genes on the paternally inherited chromosome 15q11.2-q13 region. Clinical manifestations evolve along ages, including hypothalamic dysfunction and developmental delay, cognitive disability and behavioral issues [Angulo MA, Butler MG, Cataletto ME. (2015). Prader-Willi syndrome: a review of clinical, genetic, and endocrine findings. J Endocrinol Invest, Dec;38(12):1249-63].

Angelman syndrome (AS) is a genetic disorder caused by loss of the maternal copy of the *UBE3A* gene on the 15q11-q13 chromosomal region [Maranga C, Fernandes TG, Bekman E, da Rocha ST. (2020). Angelman syndrome: a journey through the brain. FEBS J. 287(11):2154-2175]. AS is characterized by severe cognitive disability, motor dysfunction, speech impairment, hyperactivity, frequent seizures, and sleep disturbances [Margolis SS, Sell GL, Zbinden MA, Bird LM. (2015). Angelman Syndrome. Neurotherapeutics, 12(3):641-50].

Autism spectrum disorder (ASD) is a neurodevelopmental disorder that includes impairments in social communication and interaction, sensory anomalies, repetitive behaviours and varying levels of intellectual disability. ASD has a particularly large genetic contribution, and is among the most heritable common medical conditions [Lord C, Brugha TS, Charman T, Cusack J, Dumas G, Frazier T, Jones EJH, Jones RM, Pickles A, State MW, Taylor JL, Veenstra-VanderWeele J. (2020). Autism spectrum disorder. Nat Rev Dis Primers, 16;6(1):5].

Asperger syndrome (AS) is a neurodevelopmental disorder characterized by impaired social communication and interaction, average or superior intelligence, and no significant language delay. AS etiology has been linked to a variety of genetic, neurological, and environmental variables. Genomic sequencing data suggests that hundreds of genes are associated with the disorder, which are engaged in a wide range of biological processes that affect the maturation and functioning of the brain [Hosseini SA, Molla M. Asperger Syndrome. (2024) Feb 12. In: StatPearls [Internet]. Treasure Island (FL): StatPearls Publishing; 2024 Jan. Available from: https://www.ncbi.nlm.nih.gov/books/NBK557548/].

Attention deficit hyperactivity disorder (ADHD) is a nervous system condition that show patterns of developmentally inappropriate levels of inattentiveness, hyperactivity, or impulsivity. ADHD is associated with cognitive and functional deficits that relate to diffuse abnormalities in the brain [Magnus W, Nazir S, Anilkumar AC, et al. Attention Deficit Hyperactivity Disorder. [Updated 2023 Aug 8]. In: StatPearls [Internet]. Treasure Island (FL): StatPearls Publishing; 2024 Jan-. Available from: https://www.ncbi.nlm.nih.gov/books/NBK441838/].

Anxiety is mental health disorder characterized by a complex cognitive, affective, physiological, and behavioral response system associated with preparation for the anticipated events or circumstances perceived as threatening [Chand SP, Marwaha R. Anxiety. [Updated 2023 Apr 24]. In: StatPearls [Internet]. Treasure Island (FL): StatPearls Publishing; 2024 Jan-. Available from: https://www.ncbi.nlm.nih.gov/books/NBK470361/].

Depression is a mental health disorder that causes a persistent feeling of sadness and loss of interest [Chand SP, Arif H. Depression. [Updated 2023 Jul 17]. In: StatPearls [Internet]. Treasure Island (FL): StatPearls Publishing; 2024 Jan-. Available from: https://www.ncbi.nlm.nih.gov/books/NBK430847/].

Schizophrenia is a severe mental health disorder with multifactorial causes and multiple symptoms, including delusions, hallucinations, disorganized speech or behavior, and impaired cognition. [Patel KR, Cherian J, Gohil K, Atkinson D. (2014). Schizophrenia: overview and treatment options. Pharmacy and Therapeutics, 39(9):638-45]. Increased gray matter loss and aberrant network organization apparent at illness onset are associated with cognitive deficits [McCutcheon RA, Reis Marques T, Howes OD. (2020). Schizophrenia-An Overview. JAMA Psychiatry, 77(2):201-210].

Alzheimer's disease (AD) is considered the most harmful form of dementia in the elderly population, characterized by the presence of amyloid plaques in the brain, and a progressive loss of cognitive functions [Breijyeh Z, Karaman R. (2020) Comprehensive Review on Alzheimer's Disease: Causes and Treatment. Molecules. Dec 8;25(24):5789].

Huntington disease (HD) is a neurodegenerative genetic disorder originated by the elongation of cytosine, adenine, and guanine (CAG) trinucleotide repeats on the short arm of chromosome 4p16.3 in the Huntingtin (HTT) gene. This mutation leads to an abnormally long expansion of the polyglutamine in the HTT protein, which leads to neurodegeneration. The expansion also causes the HTT protein to be more prone to aggregation and accumulation that mitigates protein folding. HD is characterized by involuntary motor, cognitive and behavioral disturbances. These symptoms then progress with more cognitive deficits leading to dementia [Ajitkumar A, De Jesus O. Huntington Disease. [Updated 2023 Aug 23]. In: StatPearls [Internet]. Treasure Island (FL): StatPearls Publishing; 2024 Jan-. Available from: https://www.ncbi.nlm.nih.gov/books/NBK559166/].

Parkinson's disease (PD) is characterized by death of dopaminergic neurons in the *substantia nigra*. PD causes motor symptoms (tremor, stiffness, slowness, and imbalance), psychological or cognitive problems (cognitive decline, depression, anxiety), as well as nonmotor symptoms affecting many organ systems, such as gastrointestinal and genitourinary systems [Armstrong MJ, Okun MS. (2020). Diagnosis and Treatment of Parkinson Disease: A Review. JAMA, 323(6):548-560].

WO 2014/096377 A1 discloses hydroxyl aliphatic substituted phenyl aminoalkyl ether derivatives which are useful for the treatment of nervous system diseases, and developmental, behavioral and mental disorders associated with

cognitive deficits.

The scientific challenge to alleviate human suffering in nervous system diseases is enormous.

Thus, there is a need for new treatments of nervous system disorders associated with cognitive deficits and behavioural dysfunctions, in particular for fragile X syndrome and Rett syndrome.

## SUMMARY OF THE INVENTION

The inventors have obtained new 3-phenoxy-3-phenylpropanamine derivatives which are useful for the treatment of nervous system disorders associated with cognitive deficits and behavioural dysfunctions, in particular, fragile X syndrome and Rett syndrome. Advantageously, said compounds, which show activity in the desired nervous system disorders, do not present significant inhibition for the re-uptake of serotonin, norepinephrine nor dopamine, pharmacological activities that have been associated to modulation of nervous system disorders but with a variety of adverse events and drug-drug interactions. This is an advantage since this allows the administration of the compounds of the present invention for the treatment of nervous system disorders or its coadministration with other active compounds in the field.

Thus, in the first aspect the invention relates to a compound having formula (I)

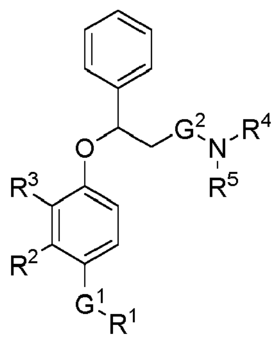

(I)

wherein,

- $R^1$ is selected from the group consisting of -OR and -NR'R", wherein R is selected from the group consisting of hydrogen, glycosyl, acyl, acetyl, and $C_1$-$C_6$ alkyl, and R' and R" are independently selected from the group consisting of hydrogen and $C_1$-$C_6$ alkyl,
- $R^2$ is selected from the group consisting of H and OH,
- $R^3$ is selected from the group consisting of H and OH,
- $R^4$ is selected from the group consisting of H, $-CH_3$ and OH,
- $R^5$ is selected from the group consisting of H and $-CH_3$,
- $G^1$ is a divalent linker comprising two carbon atoms and at least one oxygen atom selected from the group consisting of $-CH_2$-CH(OH)-, $-CH_2$-C(=O)-, -CH=C(OH)-, -CH(OH)-CH(OH)-, -CH(OH)-C(=O)-, and -C(=O)-C(=O)-, and
- $G^2$ is selected from the group consisting of $-CH_2$- and -C(=O)-,

or a pharmaceutically acceptable salt, hydrate or stereoisomer thereof.

In the second aspect, the invention relates to a pharmaceutical composition comprising a compound as defined in the first aspect and one or more pharmaceutically acceptable carriers.

In the third aspect, the invention relates to a compound as defined in the first aspect for use as a medicament.

In the fourth aspect, the invention relates to compound as defined in the first aspect for use in the treatment of nervous system disorders associated with cognitive deficits and behavioural dysfunctions.

In the fifth aspect, the invention relates to a combination comprising a compound as defined in the first aspect and another drug selected from the group consisting of drugs for the treatment of nervous system disorders or their clinical manifestations: anxiolytics and antidepressants, such as sertraline, fluoxetine, citalopram, escitalopram, trazodone, bupropion, cannabidiol, paroxetine, duloxetine, buspirone, venlafaxine, desvenlafaxine, olanzapine, mirtazapine, psilocybin and atomoxetine; antipsychotics, such as aripiprazole, risperidone, quetiapine and olanzapine; tranquilizers, such as clonazepam and lorazepam; hypnotics, such as zolpidem and melatonin; stimulants, such as methylphenidate, amphetamine, amphetamine salts and L-acetylcarnitine; non-stimulants such as clonidine and guanfacine; cognition enhancers, such as donepezil, zatolmilast, trofinetide and metformin; antiepileptics and anticonvulsants, such as levetiracetam, oxcarbazepine, valproic acid, valproate, carbamazepine, lamotrigine and topiramate; and 2-[4-[3-(methylamino)-1-phenylpropoxy]phenyl]ethanol or a pharmaceutically acceptable salt, hydrate or stereoisomer thereof.

## BRIEF DESCRIPTION OF THE FIGURES

Figure 1 shows the results of the open field test (distance travelled) in a mouse model of FXS.

Figure 2 shows the results of the open field test (movement) in a mouse model of FXS.

Figure 3 shows the results of the open field test (latency to center) in a mouse model of FXS.

Figure 4 shows the results of the three-chamber partition test in a mouse model of FXS.

Figure 5 shows the results of the novel object recognition test (exploration time) in a mouse model of FXS.

Figure 6 shows the results of the novel object recognition test (discrimination index) in a mouse model of FXS.

Figure 7 shows the results of the hyponeophagia test (latency) in a mouse model of FXS.

Figure 8 shows the results of novel object recognition (discrimination index) test in a mouse model of RS.

Figure 9 shows the results of the social recognition test in a mouse model of RS.

## DESCRIPTION OF THE INVENTION

In the first aspect the invention relates to a compound having formula (I)

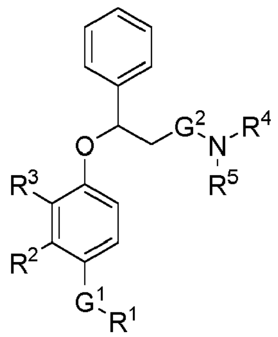

(I)

wherein,

- $R^1$ is selected from the group consisting of -OR and -NR'R", wherein R is selected from the group consisting of hydrogen, glycosyl, acyl, acetyl and $C_1$-$C_6$ alkyl, and R' and R" are independently selected from the group consisting of hydrogen and $C_1$-$C_6$ alkyl,
- $R^2$ is selected from the group consisting of H and OH,
- $R^3$ is selected from the group consisting of H and OH,
- $R^4$ is selected from the group consisting of H, $-CH_3$ and OH,
- $R^5$ is selected from the group consisting of H and $-CH_3$,
- $G^1$ is a divalent linker comprising two carbon atoms and at least one oxygen atom selected from the group consisting of $-CH_2$-CH(OH)-, $-CH_2$-C(=O)-, -CH=C(OH)-, -CH(OH)-CH(OH)-, -CH(OH)-C(=O)-, and -C(=O)-C(=O)-, and
- $G^2$ is selected from the group consisting of $-CH_2$- and -C(=O)-,

or a pharmaceutically acceptable salt, hydrate or stereoisomer thereof.

The term "alkyl" as employed herein alone or as part of another group designates both straight- and branched-chain saturated hydrocarbons containing the indicated number of carbon atoms, such as 1 to 6 carbon atoms, preferably 1

to 4 carbon atoms, more preferably 1 to 3 carbon atoms. Examples of alkyl groups are methyl, ethyl, *n*-propyl, isopropyl, *n*-butyl, isobutyl, *tert*-butyl, sec-butyl, pentyl, hexyl, isohexyl, and the various branched-chain isomers thereof.

The term "acyl" or "alkanoyl" as indistinctively used herein alone or as part of another group refers to an alkyl group attached to a carbonyl group. In the context of the present invention, the terms "acyl" and "alkanoyl" have the same meaning. Thus, a $C_1$-$C_6$ acyl or alkanoyl groups is a $C_{n-1}$ alkyl group attached to a carbonyl group. Examples of alkanoyl or acyl groups are acetyl, propionyl and butyroyl.

The term "glycosyl" as employed herein refers to a radical derived from galactose, glucose and trehalose wherein one of the hydroxyl groups, in particular the hydroxyl groups of the anomeric carbon, has been removed (to form the ether bond with the oxygen atom in the OR group). Preferably, glycosyl refers to 1-*O*-β-D-galactopyranosyl (galactose), 1-*O*-β-D-glucopyranosyl (glucose) and 1-*O*-α-D-glucopyranosyl-α-D-glucopyranosyl (trehalose).

Where the compounds of formula (I) contain acid groups (such as carboxylic acid) or basic groups (such as amines), they may form pharmaceutically acceptable salts.

As used herein, the term "pharmaceutically acceptable salt" embraces salts with a pharmaceutically acceptable acid or base. Pharmaceutically acceptable acids include both inorganic acids, for example hydrochloric, sulfuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulfonic, ethanesulfonic, benzenesulfonic, ferulic, caffeic, valproic, lipoic or p-toluenesulfonic acid. Pharmaceutical acceptable bases include alkali metal (e.g., sodium or potassium) and alkali earth metal (e. g. calcium or magnesium) hydroxides and organic bases, such as alkyl amines, arylalkyl amines and heterocyclic amines. Preferably, the pharmaceutically acceptable salt is the hydrochloride salt.

The compounds of formula (I) may also form hydrates, which are solvates of the compound of formula (I) with a water molecule via non-covalent bonding. The number of molecules compound of formula (I) with respect to the number water molecules may vary. Preferably, the hydrate is in the form of a hemihydrate, i.e. containing two molecules of the compound of formula (I) per one molecule of water.

All stereoisomers of the compounds of this invention are contemplated either alone or as mixtures thereof. The process of preparation can utilize racemates, enantiomers, or diastereomers as starting materials. When diastereomeric or enantiomeric products are prepared, they can be separated by conventional methods, for example chromatographic or functional crystallization.

The term "stereoisomer" as used herein makes reference to compounds made up of the same atoms bonded by the same sequence of bonds but having different three-dimensional structures which are not interchangeable, for example, due to the presence of chiral centers. The present invention encompasses all such variations, in particular enantiomers, diastereoisomers and mixtures thereof. Enantiomers refer to a pair of stereoisomers that differ in all stereocenters and are therefore mirror images of one another. Diastereoisomers refer to compounds that have different configurations at one or more (but not all) of the stereocenters and are not mirror images of each other.

The compounds of the present invention represented by the above-described formula (I) have at least one chiral carbon atom shown below with an asterisk *

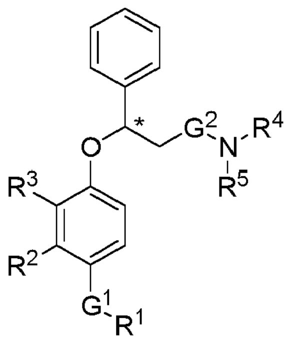

(I)

Moreover, when $G^1$ is $-CH_2-{}^*CH(OH)-$ it has a chiral carbon atom (marked with an asterisk), when $G^1$ is $-{}^*CH(OH)-C(=O)-$ it has a chiral carbon atom (marked with an asterisk), and when $G^1$ is $-{}^*CH(OH)-{}^*CH(OH)-$ it has two chiral carbon atoms (marked with an asterisk).

The present invention encompasses all stereoisomers of the compounds of formula (I), either as a single stereoisomer or mixtures of one or more stereoisomers.

$G^1$ is a divalent linker comprising two carbon atoms and at least one oxygen atom selected from the group consisting of $-CH_2-CH(OH)-$, $-CH_2-C(=O)-$, $-CH=C(OH)-$, $-CH(OH)-CH(OH)-$, $-CH(OH)-C(=O)-$, and $-C(=O)-C(=O)-$.

When $G^1$ is not symmetrical, the carbon atom depicted on the left part of the mentioned $G^1$ groups may be bonded to the benzene ring or to the $R^1$ residue; the carbon atom depicted on the right side of said $G^1$ groups may be bonded to the $R^1$ residue or the benzene ring. For example, $-CH_2-CH(OH)-$ is the same as $-CH(OH)-CH_2-$.

Preferably, in the compounds of formula (I) according to the invention, $G^1$ is selected from the group consisting of $-CH_2-C(=O)-$, $-CH(OH)-C(=O)-$ and $-C(=O)-C(=O)-$; more preferably $G^1$ is selected form the group consisting of $-CH_2-C(=O)-$, $-CH(OH)-C(=O)-$ and $-C(=O)-C(=O)-$, wherein the carbon atom depicted on the left part of said $G^1$ groups is bonded to the benzene ring and the carbon atom depicted on the right side of said $G^1$ groups is bonded to the $R^1$ residue; more preferably $G^1$ is $-CH_2-C(=O)-$ or$-C(=O)-C(=O)-$; still more preferably $G^1$ is $-CH_2-C(=O)-$ or $-C(=O)-C(=O)-$, wherein the carbon atom depicted on the left part of said $G^1$ groups is bonded to the benzene ring and the carbon atom depicted on the right side of said $G^1$ groups is bonded to the $R^1$ residue; even more preferably $G^1$ is $-CH_2-C(=O)-$; the most preferred, $G^1$ is $-CH_2-C(=O)-$ wherein the carbon atom depicted on the left part of said $G^1$ groups is bonded to the benzene ring and the carbon atom depicted on the right side of said $G^1$ groups is bonded to the $R^1$ residue.

Preferably, in the compounds of formula (I) according to the invention, at least one of $R^2$ and $R^3$ is H.

Preferably, in the compounds of formula (I) according to the invention, $R^2$ is H.

Preferably, in the compounds of formula (I) according to the invention, $R^3$ is H.

Preferably, in the compounds of formula (I) according to the invention, both $R^2$ and $R^3$ are H.

Preferably, in the compounds of formula (I) according to the invention, $R^4$ is H or $-CH_3$, more preferably H.

Preferably, in the compounds of formula (I) according to the invention, $R^5$ is $-CH_3$.

Preferably, in the compounds of formula (I) according to the invention, $G^2$ is $-CH_2-$.

Preferably, in the compounds of formula (I) according to the invention, $R^1$ is -OR; preferably -OH.

Preferably, in the compounds of formula (I) according to the invention, $R^1$ is -NR'R"; R' and R" are independently selected from the group consisting of hydrogen and $C_1$-$C_6$ alkyl; preferably hydrogen.

Preferably, in the compounds of formula (I) according to the invention, $G^1$ is selected from the group consisting of $-CH_2-C(=O)-$ and$-C(=O)-C(=O)-$, $R^1$ is -OR, $R^2$, $R^3$ and $R^4$ are H, $R^5$ is $-CH_3$ and $G^2$ is $-CH_2-$. More preferably, in the compounds of formula (I) according to the invention, $G^1$ is selected from the group consisting of $-CH_2-C(=O)-$ and $-C(=O)-C(=O)-$, wherein the carbon atom depicted on the left part of said $G^1$ groups is bonded to the benzene ring and the carbon atom depicted on the right side of said $G^1$ groups is bonded to the $R^1$ residue, $R^1$ is -OR, $R^2$, $R^3$ and $R^4$ are H, $R^5$ is $-CH_3$ and $G^2$ is $-CH_2-$.

Preferably, in the compounds of formula (I) according to the invention, $G^1$ is selected from the group consisting of $-CH_2-C(=O)-$ and $-C(=O)-C(=O)-$, $R^1$ is -OH, $R^2$, $R^3$ and $R^4$ are H, $R^5$ is $-CH_3$ and $G^2$ is $-CH_2-$. More preferably, in the compounds of formula (I) according to the invention, $G^1$ is selected from the group consisting of $-CH_2-C(=O)-$ and $-C(=O)-C(=O)-$, wherein the carbon atom depicted on the left part of said $G^1$ groups is bonded to the benzene ring and the carbon atom depicted on the right side of said $G^1$ groups is bonded to the $R^1$ residue, $R^1$ is -OH , $R^2$, $R^3$ and $R^4$ are H, $R^5$ is $-CH_3$ and $G^2$ is $-CH_2-$.

Preferably, in the compounds of formula (I) according to the invention, $G^1$ is $-CH_2-C(=O)-$, $R^1$ is -OH, $R^2$, $R^3$ and $R^4$ are H, $R^5$ is $-CH_3$ and $G^2$ is $-CH_2-$. More preferably, in the compounds of formula (I) according to the invention, $G^1$ is $-CH_2-C(=O)-$, wherein the carbon atom depicted on the left part of said $G^1$ groups is bonded to the benzene ring and the carbon atom depicted on the right side of said $G^1$ groups is bonded to the $R^1$ residue, $R^1$ is -OH, $R^2$, $R^3$ and $R^4$ are H, $R^5$ is $-CH_3$ and $G^2$ is $-CH_2-$.

Preferably, the compound of formula (I) according to the invention is selected from group consisting of:

- 2-(4-(3-(methylamino)-1-phenylpropoxy)phenyl)acetic acid
- 2-(4-(3-(methylamino)-1-phenylpropoxy)phenyl)ethane-1,1-diol
- 2-(4-(3-(methylamino)-1-phenylpropoxy)phenyl)ethene-1,1-diol
- 2-hydroxy-2-(4-(3-(methylamino)-1-phenylpropoxy)phenyl)acetic acid
- 2-(2-hydroxy-4-(3-(methylamino)-1-phenylpropoxy)phenyl)acetic acid
- 2-(3-hydroxy-4-(3-(methylamino)-1-phenylpropoxy)phenyl)acetic acid
- 2-(4-(3-(methylamino)-1-phenylpropoxy)phenyl)-2-oxoacetic acid
- 2-(4-(3-amino-1-phenylpropoxy)phenyl)ethane-1,1-diol
- 2-(4-(3-amino-1-phenylpropoxy)phenyl)acetic acid
- 2-hydroxy-1-(4-(3-(methylamino)-1-phenylpropoxy)phenyl)ethan-1-one
- methyl 2-(4-(3-(methylamino)-1-phenylpropoxy)phenyl)acetate
- 2-(4-(3-(methylamino)-1-phenylpropoxy)phenyl)acetamide

or a pharmaceutically acceptable salt, hydrate or stereoisomer thereof.

Preferably, the compound of formula (I) according to the invention is 2-(4-(3-(methylamino)-1-phenylpropoxy) phenyl)acetic acid or a pharmaceutically acceptable salt, hydrate or stereoisomer thereof; more preferably, the compound of formula (I) according to the invention is selected from group consisting of (R)-2-(4-(3-(methylamino)-1-phenylpropoxy)phenyl)acetic acid, (S)-2-(4-(3-(methylamino)-1-phenylpropoxy)phenyl)acetic acid, and mixtures thereof, preferably the racemate thereof, or a pharmaceutically acceptable salt or hydrate thereof.

More preferably, the compound of formula (I) according to the invention is 2-(4-(3-(methylamino)-1-phenylpropoxy)phenyl)acetic acid or a pharmaceutically acceptable salt, hydrate or stereoisomer thereof. Still more preferably, the compound of formula (I) according to the invention is 2-(4-(3-(methylamino)-1-phenylpropoxy)phenyl)acetic acid hydrochloride hemihydrate salt.

The compounds of formula (I) of the present invention can be prepared as shown in the following reaction schemes 1 and 2 and the description thereof. All the stereoisomers, hydrates and salts of the compounds depicted in these schemes, unless otherwise specified, are also encompassed within the scope of this invention. Protection and deprotection steps, when needed, may be carried out by general procedures (see, for example, Greene, T.W. and Wuts, P.G.M. Protecting Groups in Organic Synthesis (Third Edition), J. Wiley & Sons, 1999).

Scheme 1

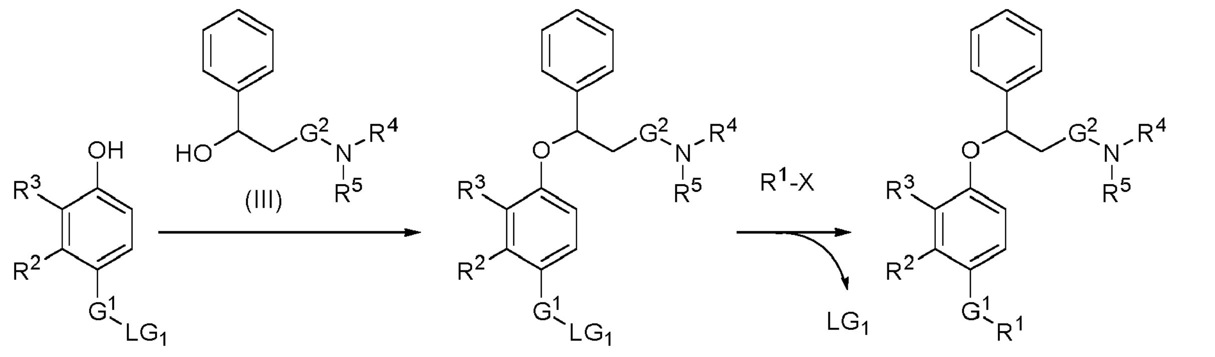


(II) (IV) (I)

The compounds of formula (I) may be synthesized by reaction of a compound of formula (II), wherein $R^2$, $R^3$ and $G^1$ are as defined for the compounds of formula (I), and $LG_1$ is a leaving group selected from halogen, alkoxy, methylsufonyl, p-toluenesulfonyl, trifluoromethylsulfonyl, and the like, with a compound of formula (III), wherein $G^2$, $R^4$ and $R^5$ are as defined for the compound of formula (I), under suitable conditions such as those of Mitsunobu reaction, to give a compound of formula (IV) wherein $R^2$-$R^5$, $G^1$, $G^2$ and $LG_1$ are as previously defined. The compound of formula (IV) may be then reacted with $R^1$-X, wherein $R^1$ is as defined for the compounds of formula (I) and X is selected from the group consisting of H and a counterion selected from the alkali and alkaline-earth metals, such as but not limited to $Li^+$, $Na^+$, $K^+$, $Cs^+$, $Mg^{2+}$, $Ca^{2+}$, and the like, under suitable acid or basic conditions, to give the compounds of formula (I).

Scheme 2

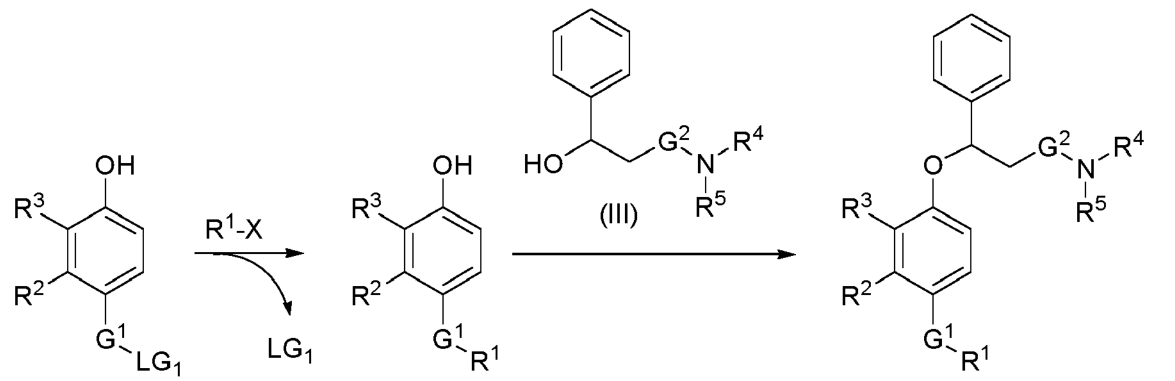


(II) (V) (I)

The compounds of formula (I) may also be synthesized by reaction of a compound of formula (II), wherein $R^2$, $R^3$ and $G^1$ are as defined for the compounds of formula (I), and $LG_1$ is a leaving group selected from halogen, alkoxy, methylsufonyl, p-toluenesulfonyl, trifluoromethylsulfonyl, and the like, with $R^1$-X, wherein $R^1$ is as defined for the

compounds of formula (I) and X is selected from the group consisting of H and a counterion selected from the alkali and alkaline-earth metals, such as but not limited to $Li^+$, $Na^+$, $K^+$, $Cs^+$, $Mg^{2+}$, $Ca^{2+}$, and the like, under suitable acid or basic conditions, to give a compound of formula (V) wherein $R^1$-$R^3$ and $G^1$ are as defined for the compounds of formula (I). The compound of formula (V) may be then reacted with a compound of formula (III), wherein $G^2$, $R^4$ and $R^5$ are as defined for the compound of formula (I), under suitable conditions such as those of Mitsunobu reaction, to give the compounds of formula (I).

Compounds of formula (II), compounds of formula (III), and $R^1$-X are either commercially available of may be prepared by conventional procedures known in the art.

The term "halogen" as used herein alone or as part of another group refers to chlorine, bromine, fluorine and iodine.

The term "alkoxy" as employed herein alone or as part of another group designates an alkyl group containing 1 to 20 carbons, preferably 1 to 10 carbons, more preferably 1 to 8 carbons, more preferably 1 to 6 carbons, and still more preferably 1 to 3 carbons linked to an oxygen atom. Examples of alkoxy groups are methoxy, ethoxy, n-propoxy, isopropoxy, n-butoxy, isobutoxy, tert-butoxy, pentoxy, hexyloxy, isohexyloxy, heptyloxy, 4,4-dimethylpentoxy, octyloxy, 2,2,4-trimethylpentoxy, nonyloxy, decyloxyl, undecyloxy, dodecyloxy, and the various branched-chain isomers thereof.

In a second aspect, the invention relates to a pharmaceutical composition comprising a compound as defined in the first aspect and one or more pharmaceutically acceptable carrier.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government, such as the ones from Europe, USA and Japan, or listed in the EU, U.S. or Japan Pharmacopeia or other generally recognized pharmacopeia for use in animals, and more particularly in humans.

The term "carrier" refers to a diluent, adjuvant, excipient, vehicle or mixture thereof with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

Examples of pharmaceutical compositions include any solid (tablets, pills, capsules, granules, etc.) or liquid (solutions, suspensions or emulsions) composition for oral, intranasal, topical or parenteral administration, such as concentrate, dispersion, drops, emulsion, liquid, solution, suspension, spray, syrup, cream, gel, ointment, paste, block, sachet, cachet, capsule, film, granules, gum, lozenge, lyophilizate, pastille, patch, pellets, pessary, plaster, powder, stick, suppository, tablets and mini-tablets. The pharmaceutical composition can be formulated employing carriers according to the desired mode of administration, under conventional, delayed, modified and prolonged release characteristics.

Preferably, pharmaceutical compositions for parenteral administration may comprise physiologically acceptable sterile aqueous or non-aqueous solutions, emulsions, suspensions (in particular in the case of intramuscular administration or implants) and powders for reconstitution as injectable dosage forms.

Preferably, pharmaceutical compositions for parenteral administration may also comprise the compounds of the invention of formula (I) in powder form which may be diluted in phosphate buffered saline comprising polysorbate 80, potassium chloride, monobasic phosphate, sodium chloride, dibasic sodium phosphate and water for injection prepared to the desired proportion and volume.

Preferably, liposome injections for intrathecal use may be prepared in a sterile, injectable dispersion of compounds of formula (I), encapsulated in multi-vesicular lipid-based particles.

Preferably, pharmaceutical compositions for oral administration may be in the form of tables, capsules or liquid compositions such as aqueous based oral solutions and oral suspensions.

Tablets may be prepared containing as inactive ingredients lactose, magnesium stearate, hydroxypropylmethylcellulose, polyethylene glycol, povidone, sodium starch glycolate and titanium dioxide.

Capsules may be prepared containing as inactive ingredients talcum, sodium lauryl sulfate, colloidal silicon dioxide, magnesium stearate, titanium dioxide (E171), hard gelatin or hydroxypropylmethylcellulose capsule, black ink, propylene glycol and shellac.

Aqueous based oral solutions may be prepared containing as inactive ingredients citric acid, sodium citrate, benzoic acid, fruit flavorings, hydroxyethylcellulose, sorbitol, and water.

Oral suspensions may be prepared containing as inactive ingredients glycerin, carboxymethylcellulose sodium, hydroxyethylcellulose, citric acid, sodium citrate, benzoic acid, simethicone, polysorbate 80, flavorings, and water.

As shown in the examples, the compounds of formula (I) are useful for the treatment of nervous system disorder associated with cognitive deficits and behavioural dysfunctions, in particular fragile X syndrome and Rett syndrome.

Thus, in a third aspect, the invention relates to a compound as defined in the first aspect for use as a medicament.

In a fourth aspect, the invention relates to a compound as defined in the first aspect for use in the treatment of, preferably wherein the nervous system disorder associated with cognitive deficits and behavioural dysfunctions is selected from the group consisting of fragile X syndrome, Rett syndrome, Down syndrome, DiGeorge syndrome (22q11.2 deletion syndrome), Prader-Willi syndrome, Angelman syndrome, autism spectrum disorder, Asperger syndrome, attention deficit hyperactivity disorder, anxiety, depression, schizophrenia, Alzheimer's disease, Huntington's disease, Parkinson's disease; more preferably Rett syndrome; still more preferably fragile X syndrome.

This aspect may be also formulated as the use of a compound as defined in the first aspect in the manufacture of a medicament for the treatment of, preferably wherein the nervous system disorder associated with cognitive deficits and behavioural dysfunctions is selected from the group consisting of fragile X syndrome, Rett syndrome, Down syndrome, DiGeorge syndrome (22q11.2 deletion syndrome), Prader-Willi syndrome, Angelman syndrome, autism spectrum disorder, Asperger syndrome, attention deficit hyperactivity disorder, anxiety, depression, schizophrenia, Alzheimer's disease, Huntington's disease, Parkinson's disease; more preferably fragile X syndrome or Rett syndrome; more preferably Rett syndrome; still more preferably fragile X syndrome.

This aspect may be also formulated as the method of treatment of nervous system disorders associated with cognitive deficits and behavioural dysfunctions, preferably wherein the nervous system disorder associated with cognitive deficits and behavioural dysfunctions is selected from the group consisting of fragile X syndrome, Rett syndrome, Down syndrome, DiGeorge syndrome (22q11.2 deletion syndrome), Prader-Willi syndrome, Angelman syndrome, autism spectrum disorder, Asperger syndrome, attention deficit hyperactivity disorder, anxiety, depression, schizophrenia, Alzheimer's disease, Huntington's disease, Parkinson's disease; more preferably fragile X syndrome or Rett syndrome, more preferably Rett syndrome, still more preferably fragile X syndrome; wherein the method comprises administering a compound as defined in the first aspect to a subject in need thereof.

The term treatment is used to designate the administration of a pharmaceutically active compound or a composition thereof to control disease progression before or after the clinical signs have appeared. By control of the disease progression, it is meant to designate beneficial or desired clinical results including, but not limited to, reduction of symptoms, reduction of the length of the disease, stabilization of the pathological state (specifically avoidance of further deterioration), delay in the disease progression, improvement of the pathological state and remission (both partial and total). Control of disease progression can also entail prolonged survival, compared to the expected survival if the treatment is not applied. In a particular embodiment of the invention, the compounds and compositions of the invention may be used to control the disease progression once at least one of the clinical signs of the disease has appeared.

The expression "nervous system disorder associated with cognitive deficits and behavioural dysfunctions" refers to developmental anomalies, such as fragile X syndrome, Rett syndrome, Down syndrome, DiGeorge syndrome (22q11.2 deletion syndrome), Prader-Willi syndrome, and Angelman syndrome; mental, behavioural and neurodevelopmental disorders such as autism spectrum disorder, Asperger syndrome, attention deficit hyperactivity disorder, anxiety, depression, and schizophrenia; and diseases of the nervous system, such as Alzheimer's disease, Huntington's disease, and Parkinson's disease; as defined by the World Health Organization in the International Classification of Diseases 11th Revision (ICD-11, https://icd.who.int/).

The compounds of the first aspect may also be administered in combination with other drugs that are also useful for the treatment of nervous system disorders associated with cognitive deficits and behavioural dysfunctions.

Thus, in the fifth aspect, the invention relates to a combination comprising a compound as defined in the first aspect and another drug selected from the group consisting of drugs for the treatment of nervous system disorders or their clinical manifestations: anxiolytics and antidepressants, such as sertraline, fluoxetine, citalopram, escitalopram, trazodone, bupropion, cannabidiol, paroxetine, duloxetine, buspirone, venlafaxine, desvenlafaxine, olanzapine, mirtazapine, psilocybin and atomoxetine; antipsychotics, such as aripiprazole, risperidone, quetiapine and olanzapine; tranquilizers, such as clonazepam and lorazepam; hypnotics, such as zolpidem and melatonin; stimulants, such as methylphenidate, amphetamine, amphetamine salts and L-acetylcarnitine; non-stimulants, such as clonidine and guanfacine; cognition enhancers, such as donepezil, zatolmilast, trofinetide and metformin; antiepileptics and anticonvulsants, such as levetiracetam, oxcarbazepine, valproic acid, valproate, carbamazepine, lamotrigine and topiramate; and 2-[4-[3-(methylamino)-1-phenylpropoxy]phenyl]ethanol or a pharmaceutically acceptable salt, hydrate or stereoisomer thereof.

The term "combination" refers to a one or more compositions wherein the above-mentioned products are distributed among the one or more compositions, for example one or two compositions, in particular wherein one composition comprises the compound of formula (I) as defined in the first aspect and another composition comprises the other drug, or one composition comprising all the compounds.

In a particular embodiment, the combination does not comprise 2-[4-[3-(methylamino)-1-phenylpropoxy]phenyl] ethanol or a pharmaceutically acceptable salt, hydrate or stereoisomer thereof.

In a particular embodiment, the combination comprises 2-[4-[3-(methylamino)-1-phenylpropoxy]phenyl]ethanol or a pharmaceutically acceptable salt or stereoisomer thereof and 2-(4-(3-(methylamino)-1-phenylpropoxy)phenyl)acetic acid or a pharmaceutically acceptable salt, hydrate or stereoisomer thereof.

Preferably, the 2-[4-[3-(methylamino)-1-phenylpropoxy]phenyl]ethanol or a pharmaceutically acceptable salt or

stereoisomer thereof is in the form of the hydrochloride salt. In one embodiment, the 2-[4-[3-(methylamino)-1-phenylpropoxy]phenyl]ethanol or a pharmaceutically acceptable salt or stereoisomer thereof is in the form of the (S)-2-[4-[3-(methylamino)-1-phenylpropoxy]phenyl]ethanol enantiomer, in the form of the (R)-2-[4-[3-(methylamino)-1-phenylpropoxy]phenyl]ethanol enantiomer, or mixtures thereof. More preferably, the (S)-2-[4-[3-(methylamino)-1-phenylpropoxy] phenyl]ethanol or a pharmaceutically acceptable salt or stereoisomer thereof is in the form of (S)-2-[4-[3-(Methylamino)-1 - phenylpropoxy]phenyl]ethanol hydrochloride, in the form of (R)-2-[4-[3-(Methylamino)-1 -phenylpropoxy]phenyl]ethanol hydrochloride, or mixtures thereof.

Drugs of the present invention may be administered as a liquid, semi-solid or solid state of matter. Any dosage form commonly used for drugs, such as concentrate, dispersion, drops, emulsion, liquid, solution, suspension, spray, syrup, cream, gel, ointment, paste, block, sachet, cachet, capsule, film, granules, gum, lozenge, lyophilizate, pastille, patch, pellets, pessary, plaster, powder, stick, suppository, and tablets, may be used. The pharmaceutical composition can be formulated employing conventional carriers according to the desired mode of administration, under conventional, delayed, modified and prolonged release characteristics. The intended site may be buccal, endocervical, gastric, gastroenteral, intestinal, intraarticular, intraperitoneal, intrathecal, intrauterine, intravesical, nasal, oral, parenteral, pulmonary, rectal, sublingual, subcutaneous, topical, transdermal, urethral or vaginal by swallowing, chewing, sucking application, bioadhesion, insertion, orodispersion, infusion, inhalation, and injection administration methods. Preferably, the compositions of the invention are administered orally (solids or liquids), transdermally, subcutaneously, parenterally, locally (ointments, creams, gels, plasters, powders), as drops or as a nasal or buccal compositions.

When the combination as defined above comprises more than one composition, for example two compositions as defined above, said compositions may be administered simultaneously or separately.

Simultaneous administration refers to the administration of the compositions at the same time point, for example within a time span of no more than 5 minutes. Simultaneous administration is feasible for combinations as defined above in the form of one or more compositions, such as one or two compositions.

Separate administration refers to the administration of one composition at one time point and another composition at another time point independently of each other at different time points, for example wherein the time points are separated by more than 5 minutes, such as more than 15 minutes, more than 1 hour, more than 2 hours, more than 3 hours or more than 6 hours. Separate administration is only feasible when the combination as defined above is in the form of more than one composition, such as two compositions.

## EXAMPLES

The following examples represent specific embodiments of the present invention.

### Example 1. Synthesis of 2-(4-(3-(methylamino)-1-phenylpropoxy)phenyl)acetic acid

Methyl 2-(4-3-((tert-butoxycarbonyl)(methyl)amino)-1-phenylpropoxy)phenyl) acetate

Method A:

Triphenylphosphine (1.25 eq.), methyl 2-(4-hydroxy-phenyl)acetate (1.0 eq.) and tert-butyl 3-hydroxy-3-phenylpropyl(methyl)carbamate (1.27 eq.) were dissolved in toluene under nitrogen and cooled to 0 °C. A solution of DEAD (-40% in toluene, 1.25 eq.) was added drop wise at 0°C. The mixture was allowed to warm to rt during 1h and heated at 80 °C for 21 h. Then, it was cooled to room temperature, diluted with ethyl acetate and washed with 5% HCl (3x), dried over anhydrous MgSO4 and concentrated under vacuum. The resulting crude was purified by flash chromatography to afford the expected product in 74% yield as a colorless oil.

Method B:

Methyl 2-(4-hydroxy-phenyl)acetate (1.0 eq.), tert-butyl 3-hydroxy-3-phenylpropyl(methyl)carbamate (1.1 eq.) and Triphenylphosphine (1.2 eq.) was dissolved Toluene and cooled to 0-10°C. A 40% DEAD solution in toluene (1.2 eq.) was successively added at 0-10°C and the mixture was stirred at 0-10 °C for 1 h and allowed the reaction mass temperature to reach 25-35°C. Progress of the reaction was monitored by HPLC/TLC. After completion of the reaction, add *n*-Heptane at 25-35 °C. Allow the reaction mass to reach 0-10 °C and stir the reaction mixture at 0-5°C for 1-2 h. Filter the reaction mass and wash the wet cake with 25% toluene in heptane mixture. Filtered liquid was washed with water and brine. The organic layer was dried over anhydrous $Na_2SO_4$ and concentrated the solvent up to 2.5-3 vol. and co-distilled with n-hexanes. The crude obtained was purified by silica gel column chromatography to afford the expected product in 50% yield.

2-(4-3-((tert-butoxycarbonyl)(methyl)amino)-1-phenylpropoxy)phenyl)acetic acid

To a stirred solution of methyl 2-(4-3-((tert-butoxycarbonyl)(methyl)amino)-1-phenylpropoxy)phenyl) acetate (1.0 eq.) in THF (10 vol.) and water (2.0 vol.) at room temperature, $LiOH \cdot H_2O$ (2.5 eq.) was added and the mixture was stirred for 2h at room temperature. Reaction progress was monitored by TLC. After completion of reaction, reaction mass was distilled to remove the THF solvent. Water (10 vol.) was added to the reaction mass and pH adjusted to 2-3 with 6N HCl at 10-15 °C. Reaction mass was extracted with Ethyl acetate at room temperature. Distilled off the Ethyl acetate solvent up to 4.0-5.0 vol. remains in the reaction mass. Filtered the reaction mass at room temperature and dried the material at 50-55 °C. Ethyl acetate was added to the dried material and heated the mass at 50-55 °C and stirred for 20 min. Cooled the reaction mass to 10-15 °C and 4N HCl in Ethyl acetate was added to the reaction mass and stirred for 2 h at 10-15 °C. Reaction progress was monitored by TLC. After completion of reaction, reaction mass was filtered, washed ethyl acetate and the material was dried at 50-55 °C.

2-(4-3-(methylamino)-1-phenylpropoxy)phenyl)acetic acid hydrochloride hemihydrate

To a stirred solution of methyl 2-(4-3-((tert-butoxycarbonyl)(methyl)amino)-1-phenylpropoxy)phenyl) acetate (1.0 eq.) in THF and water at room temperature, $LiOH \cdot H_2O$ was added and stirred for 3h at room temperature. Reaction progress was monitored by TLC. After completion of reaction, reaction mass was distilled to remove the THF solvent. Water was added to the reaction mass and pH adjusted to 2-3 with 6N HCl at 10-15 °C. Reaction mass was extracted with ethyl acetate at room temperature. The aqueous and organic layers were separated and the organic layer was worked-up in brine, filtered and the solvent distilled to dryness. Ethyl acetate was added to the dried material and heated the mass at 50-55 °C and stirred for 20 min. Cooled the reaction mass to 10-15 °C and 4N HCl in ethyl acetate was added to the reaction mass and stirred for 2 h at 10-15 °C. After completion of reaction, crude was filtered, washed with ethyl acetate and dried to afford 2-(4-(3-(methylamino)-1-phenylpropoxy)phenyl)acetic acid hydrochloride hemihydrate (Compound 1) in 67% yield.

$^1H$ NMR (400 MHz, DMSO-$d_6$), δ: 9.42 (br, 1H), 7.42 (d, $J$ = 6.8, 2H), 7.35 (t, $J$ = 7.2, 2H), 7.26 (t, $J$ = 7.2 Hz, 1H), 7.08 (d, $J$ = 8.8 Hz, 2H), 6.86 (d, $J$ = 8.4 Hz, 2H), 5.58-5.55 (m, 1H), 3.44 (s, 2H), 3.04 - 2.98 (m, 2H), 2.52 (s, 3H), 2.32 - 2.19 (m, 2H) ppm.

$^{13}C$ NMR (100 MHz, DMSO-$d_6$), δ: 172.93, 156.04, 140.84, 130.32, 128.71, 127.88, 127.46, 126.04, 115.74, 76.14, 45.28, 40.12, 34.24, 32.37 ppm.

Elemental analysis: Found: C, 63.12%; H, 6.77%; N, 4.09%; O, 16.19%; S, 0.00%.

Cl content (by potentiometric analysis): 10.54%
$H_2O$ content (by Karl-Fisher analysis): 2.2%
Molecular formula: $C_{18}H_{21}NO_3 \cdot HCl \cdot ½H_2O$
Molecular weight: 344.82 g/mol
MS-ESI (+): (m/z): $[M+H]^+$= 300.15

**Example 2. Synthesis of 2-hydroxy-2-(4-(3-(methylamino)-1-phenylpropoxy)phenyl)acetic acid**

Title compound was prepared in moderate to low yield following the same procedure described in Example 1, using methyl 2-hydroxy-2-(4-hydroxyphenyl)acetate as starting material.
MS-ESI (+): (m/z): $[M+H]^+$= 316.15

**Example 3. Synthesis of 2-(2-hydroxy-4-(3-(methylamino)-1-phenylpropoxy)phenyl)acetic acid**

Title compound was prepared in moderate to low yield following the same procedure described in Example 1, using methyl 2,4-dihydroxyphenylacetate as starting material.
MS-ESI (+): (m/z): $[M+H]^+$= 316.15

**Example 4. Synthesis of 2-(3-hydroxy-4-(3-(methylamino)-1-phenylpropoxy)phenyl)acetic acid**

Title compound was prepared in moderate to low yield following the same procedure described in Example 1, using methyl 3,4-dihydroxyphenylacetic acid as starting material.
MS-ESI (+): (m/z): $[M+H]^+$= 316.15

**Example 5. *In vitro* pharmacology**

### 5.1. Materials and methods

#### 5.1.1. Binding assays

The *in vitro* binding activity to the norepinephrine transporter was tested following the experimental protocol disclosed in Pacholczyk, T. [Nature, (1991), 350: 350-354] on human recombinant CHO cells using [$^3$H]nisoxetine as the ligand at 1 nM concentration ($K_d$ 2.9 nM), the non-specific binding was determined using desipramine (1 μM), the incubation time was 120 min at 4 °C and the detection method was by scintillation counting.

The *in vitro* binding activity to the dopamine transporter was tested following the experimental protocol disclosed in Pristupa, Z.B. et al. [Mol. Pharmacol., (1994), 45: 125-135] on human recombinant CHO cells using [$^3$H]BTCP as the ligand at 4 nM concentration ($K_d$ 4.5 nM), the non-specific binding was determined using BTCP (10 μM), the incubation time was 120 min at 4 °C and the detection method was by scintillation counting.

The *in vitro* binding activity to the 5-HT transporter was tested following the experimental protocol disclosed in Tatsumi, M. et al. [Eur. J. Pharmacol., (1999), 368: 277-283] on human recombinant CHO cells using [$^3$H]imipramine as the ligand at 2 nM concentration ($K_d$ 1.7 nM), the non-specific binding was determined using imipramine (10 μM), the incubation time was 60 min at room temperature and the detection method was by scintillation counting.

Compounds were tested at 1.0E-05 M. Compound binding was calculated as a % inhibition of the binding of a ligand specific for each target.

#### 5.1.2. Enzyme and uptake assays

The *in vitro* uptake by the norepinephrine transporter was tested following the experimental protocol disclosed in Perovic, S. and Muller, W.E.G. [Arzneim-Forsch. Drug Res., (1995), 45:1145-1148] on human recombinant cells using norepinephrine hydrochloride, DL-[7-3H(N)] (500 nM) as the substrate, the incubation time was 180 min at room temperature, measuring [3H]NE incorporation into cells by scintillation counting.

The *in vitro* uptake by the dopamine transporter was tested following the experimental protocol disclosed in Verrico C. et al. [Psychopharmacology (Berl), (2005), in press] on human recombinant cells using 3H DA (300 nM) / DA (300 nM) as the substrate, the incubation time was 90 min at room temperature, measuring [3H]DA incorporation into cells by scintillation counting.

The *in vitro* uptake by the serotonin transporter was tested following the experimental protocol disclosed in Verrico C. et al. [Psychopharmacology (Berl), (2005), in press] on human recombinant cells using 5HT (150 nM) / 3H 5HT as the substrate, the incubation time was 120 min at room temperature, measuring [3H]5HT incorporation into cells by scintillation counting.

### 5.2. Results

The results are expressed as a percent of control specific binding:

$$\frac{\text{measured specific binding}}{\text{control specific binding}} * 100$$

and as a percent inhibition of control specific binding:

$$100 - \left(\frac{\text{measured specific binding}}{\text{control specific binding}} * 100\right)$$

obtained in the presence of the test compound.

Results showing an inhibition higher than 50% are considered to represent significant effects of the test compounds. Results showing an inhibition (or stimulation) between 25% and 50% are indicative of weak to moderate effects (they should be confirmed by further testing as they are within a range where more inter-experimental variability can occur). Results showing an inhibition lower than 25% are not considered significant and mostly attributable to variability of the signal around the control level.

The $IC_{50}$ values (concentration causing a half-maximal inhibition of control specific binding) and Hill coefficients (nH) were determined by non-linear regression analysis of the competition curves generated with mean replicate values using Hill equation curve fitting

$$Y = D + \left[\frac{A - D}{1 + (\frac{C}{C_{50}})^{nH}}\right]$$

where Y = specific binding, A = left asymptote of the curve, D = right asymptote of the curve, C = compound concentration, $C_{50}$ = $IC_{50}$, and nH = slope factor. This analysis was performed using software developed at Cerep (Hill software) and validated by comparison with data generated by the commercial software SigmaPlot® 4.0 for Windows® (© 1997 by SPSS Inc.). The inhibition constants (Ki) were calculated using the Cheng Prusoff equation

$$K_i = \frac{IC_{50}}{(1 - L/K_D)}$$

where L = concentration of ligand in the assay, and $K_D$ = affinity of the ligand for the receptor.

The reference compound parameters for each assay are provided in the tables below:

Table 1

| *Binding assay* | *Reference compound* | *$IC_{50}$ (M)* | *Ki (M)* |
|---|---|---|---|
| Serotonin (5-HT) transporter (h) (antagonist radioligand) | imipramine | 4.1E-09 M | 1.9E-09 M |
| Norepinephrine transporter (h) (antagonist radioligand) | protriptyline | 5.4E-09 M | 4.0E-09 M |
| Dopamine transporter (h) (antagonist radioligand) | BTCP | 1.4E-08 M | 7.4E-09 M |

Table 2

| *Enzyme and uptake assay* | *Reference compound* | *$IC_{50}$ (M)* | *nH* |
|---|---|---|---|
| Serotonin (5-HT) transporter uptake (h) | imipramine | 1.2E-08 M | n/a |
| Norepinephrine transporter uptake (h) | protriptyline | 4.3E-10 M | n/a |
| Dopamine transporter uptake (h) | GBR 12909 | 1.6E-09 M | n/a |

### 5.2.1. Binding assay

The results of the binding assays of Compound 1 are gathered in the table below:

Table 3

| *Binding assay* | *% Inhibition of control specific binding* | | |
|---|---|---|---|
| | 1st | 2nd | Mean |
| Serotonin (5-HT) transporter (h) (antagonist radioligand) | 31.0 | 26.4 | 28.7 |
| Norepinephrine transporter (h) (antagonist radioligand) | 3.8 | 4.5 | 4.2 |
| Dopamine transporter (h) (antagonist radioligand) | 4.6 | 1.0 | 2.8 |

Results showing an inhibition lower than 25% are not considered significant and mostly attributable to variability of the signal around the control level. Results showing an inhibition (or stimulation) between 25% and 50% are indicative of weak to moderate effects (they should be confirmed by further testing as they are within a range where more inter-experimental variability can occur). Particularly, further testing of serotonin transporter uptake to confirm the above findings is disclosed in the next section.

### 5.2.2. Enzyme and uptake assays

The results of the $IC_{50}$ determination for serotonin, norepinephrine and dopamine transporter of Compound 1 is gathered in the table below:

Table 4

| *Enzyme and uptake assay* | *$IC_{50}$ (M)* |
|---|---|
| Serotonin (5-HT) transporter uptake (h) | > 1.0E-04 |
| Norepinephrine transporter uptake(h) | > 1.0E-05 |
| Dopamine transporter uptake (h) | > 1.0E-04 |

$IC_{50}$ value above the highest test concentration. Concentration-response curve shows less than 50 % effect at the highest validated testing concentration.

Therefore, there was no significant effect of Compound 1 in any of the assays, either binding and enzyme and uptake assays, in the selected targets.

In conclusion, compounds of the present invention do not present significant inhibition for the re-uptake of serotonin, norepinephrine nor dopamine, thus preventing potential adverse events and drug-drug interactions associated to this pharmacological activity.

**Example 6. Evaluation of the therapeutic activity in a mouse model of Fragile X Syndrome (FXS)**

### 6.1. Fragile X syndrome (FXS) mouse model

In this study, we used the FXS mouse model, Fragile X messenger ribonucleoprotein 1 KO2 *(Fmr1* KO2) and their corresponding wildtype (WT) littermates.

*Fmr1* KO2 mice were generated by deleting the promoter and first exon of the *fmr1* gene according to Mientjes *et al.* [Mientjes E. J., Nieuwenhuizen I., Kirkpatrick L., Zu T., Hoogeveen-Westerveld M., Severijnen L., et al. (2006). The generation of a conditional Fmr1 knock out mouse model to study Fmrp function in vivo. Neurobiol. Dis., 21, 549-555] and then were backcrossed to a C57BL/6J background for more than eight generations. The *Fmr1* KO2 is both, protein and mRNA null, and recapitulate behavioral symptoms observed in human FXS pathology, including hyperactivity, repetitive behavior, and deficits in learning and memory [Gaudissard J, Ginger M, Premoli M, Memo M, Frick A, Pietropaolo S. (2017). Behavioral abnormalities in the Fmr1-KO2 mouse model of fragile X syndrome: The relevance of early life phases. Autism Res.,10(10):1584-1596].

For dosing, each drug/vehicle was administered on the lower abdominal quadrant via the i.p. route.

Following pretreatment, all mice continued their dosing regimen, outlined in Table 5, until the completion of all the behavioral phenotyping. On the day of behavioral testing, mice were dosed before the start of the behavioral assay.

### 6.2. Dosing regimen

Three control groups of 15 mice each were utilized for this study. WT and *Fmr1* KO2 control groups (Group 1 and 2) were administered vehicle in an identical manner and volume to that utilized for the treated *Fmr1* KO2 group. WT drug control (Group 3) was administered the test compound (Compound 1) in an identical manner and volume to that utilized for the treated *Fmr1* KO2 group. One treated *Fmr1* KO2 group of 10 mice was utilized for this study (Group 4).

Table 5

| Group | Drugs | Drug Regimen | Dose (mg/kg) | N Animals |
|---|---|---|---|---|
| 1 | WT-Saline | i.p. | - | 15 |
| 2 | *Fmr1* KO2-Saline | i.p. | - | 15 |
| 3 | WT-Test compound | i.p. | 30 | 15 |
| 4 | *Fmr1* KO2-Test compound | i.p. | 30 | 15 |

The administration volume was 0.1 mL /10 g of body weight.

### 6.3. Cognition and Behavior assays

Cognition and behavior tests were separated by a minimum of 3 days, during which the mice were dosed with vehicle and compound.

1) Behaviour - Hyperactivity: Open field test
2) Behaviour - Sociability: Three chamber partition test
3) Cognition - Memory and Learning: Novel Object Recognition test
4) Behaviour - Anxiety: Hyponeophagia test

Each cognition and behavior test were performed between 8 a.m. and 4 p.m. Mice were dosed in the housing room (prior to testing, see administration details in each behavioral test in the Methods section) and then brought to the experimental room to acclimate for 20 min before testing. Animals were tested in only one behavioral task on each experimental day, and each additional behavioral test was separated by at least 3 days.

Prior to each test, a mouse that was not included in the study was placed in the experimental apparatus for 3 min. Then, this non-study animal was removed, and the apparatus was cleaned with moist and dry tissues before placing a study mouse into the apparatus. The aim was to create a low but constant background mouse odour for all experimental subjects.

Experimenters were blinded to mouse genotype and treatment throughout all behavioural tests and data analysis.

### 6.4. Methods

#### 6.4.1. Animal housing

The *Fmr1* KO2 mice were housed in four per cage groups of the same genotype in a temperature- and humidity-controlled room with a 12-h light-dark cycle (lights on 7 a.m.-7 p.m.). Mice were housed in commercial plastic cages (40 × 23 × 12 cm) with Aspen bedding and without environmental enrichment on a ventilated rack system. Food and water were available *ad libitum,* except during test sessions. Testing was conducted during the light phase on male *Fmr1* KO2 mice and their WT littermates.

#### 6.4.2. Open-field hyperactivity

An open-field apparatus [Olmos-Serrano J. L., Corbin J. G., Burns M. P. (2011). The GABAA receptor agonist THIP ameliorates specific behavioral deficits in the mouse model of fragile X syndrome. Dev. Neurosci., 33, 395-403] was used to test hyperactivity and habituation to a novel environment, in which decreased exploration as a function of repeated exposure to the same environment may be an index of memory. Each mouse was exposed individually to the open field in one session corresponding to 60-min post-test article administration. The open-field assay was performed using an automated system including a Noldus activity monitor chamber with the associated EthoVision software (Noldus Information Technology Inc., Leesburg, VA, USA). A mouse was placed into a corner square facing the wall and horizontal locomotor activity, measured as distance travelled in centimeters (cm) by the number of squares entered with the whole body, was recorded for 30 min.

#### 6.4.3. Social recognition

In the three-chambered social novelty task, a subject mouse was evaluated for its preference to explore a novel versus a familiar social stimulus mouse, defined as the time spent in the chamber with the novel mouse versus the chamber with the familiar mouse. The apparatus was a rectangular three-chambered box, in which each chamber measured 20 cm (length) × 40.5 cm (width) × 22 cm (height) (Spectrum Diversified Designs, Inc., Streetsboro, OH, USA). During the sociability phase, the mouse was allowed to freely explore all three chambers, in which one side chamber contained a mouse and the other side contained a mouse for 10 min. During the social novelty phase, the mouse was allowed to freely explore all three chambers, in which one side chamber still contained a familiar mouse (f) and the other side chamber now contained a novel mouse (n) for 10 min. The novel mouse was enclosed in a wire cage identical to that enclosing the familiar mouse. For each phase of the test, the amount of time spent in each chamber was recorded. An entry was defined as all four paws in one chamber. Test article administration was performed just after the exposure and recognition of the familiar stimuli mice.

#### 6.4.4. Novel object recognition

Recognition memory of a familiar object compared to a novel object was assessed by the novel object recognition (NOR) task. A Plexiglas box (26 cm length × 20 cm width × 16 cm height) and two unique objects (4-6 cm diameter × 2-6 cm height), each in duplicate, were used. Mice were habituated individually to the experimental environment by allowing them to freely explore the box, which was empty, for 20 min per day for two consecutive days before testing. The test

involved two consecutive trials, each 5 min in duration. For trial one, two identical objects were placed in the box, and the mouse was allowed to freely explore the objects for 5 min. These objects would be the familiar (f) objects. For trial two, one familiar object (f) is replaced with one novel object (n), and the mouse is allocated 5 min to explore.

Object exploration was defined as the mouse sniffing or touching the object with its nose, vibrissa, mouth or forepaws. Time spent near or standing on top of the objects without interacting with the object was not counted as exploration. Test article administration was administered 60 minutes prior to the novel object recognition assay.

6.4.5. Hyponeophagia or novelty-suppressed feeding

The novelty-suppressed feeding test, in which a highly palatable but novel liquid food was available for consumption in a novel environment, measured the latency to consume a defined amount of the novel food as an index of anxiety-like behaviour. Mice were food restricted overnight and tested the next morning. Twenty minutes prior to the test, each mouse was individually placed into a temporary holding cage to prevent social transmission of food preferences. Test article administration was performed 60 minutes prior to the exposure to new food type. Testing was conducted in a chamber (30 cm length $\times$ 30 cm width $\times$ 5 cm height) with three white walls and a fourth wall of transparent plastic to allow observation of the mouse. A food well (1.2 cm diameter, 0.9 cm height) was glued to the white Perspex base of the test chamber. An individual mouse was placed into the chamber facing away from the food well containing sweetened condensed milk diluted 50:50 with water. The latency from placement in the test chamber to the start of a proper drinking bout, defined as drinking continuously was measured.

6.4.6. Statistical analysis

All statistical analysis and graphs were performed using GraphPad Prism software (GraphPad Software) where each point represents data from an individual mouse.

**6.5. Results**

6.5.1. Hyperactivity: open field test

The effects of single administration of vehicle or Compound 1 (30 mg/kg, i.p.) on hyperactivity are presented in Figure 1 (total distance travelled), Figure 2 (movement), and Figure 3 (latency to center).

• *Distance travelled:*

The results are shown in Figure 1. Vehicle-treated *Fmr1* KO2 mice display a hyperactive phenotype by travelling a significantly greater distance in the open field compared to WT mice. Treatment with Compound 1 at 30 mg/kg significantly reduced locomotor activity back to levels observed in the WT vehicle group. Bars indicate mean values (mean $\pm$ SEM). Points correspond to values from individual mice. Asterisks represent significant change; ns, not significant; ****P $< 0.0001$. n = 15.

• *Movement:*

The results are shown in Figure 2. Only two spatial measures are collected in this test: the total distance travelled (considered as a measure of general activity) and some measure of the animal's tendency to avoid the arena center. Movement time represents the time spent moving. We evaluated the effect of Compound 1 (30 mg/kg) treatment in *Fmr1* KO2 mice movement in the open field test. *Fmr1* KO2 mice treated with Compound 1 (30 mg/kg) show a significant correction of total movement when compared to WT littermates treated with vehicle (Compound 1 (30 mg/kg), p = 0.1337). Bars indicate mean values (mean $\pm$ SEM). Points correspond to values from individual mice. Asterisks represent significant change; ns, not significant; ****P $< 0.0001$. n = 15.

• *Latency to center.*

The results are shown in Figure 3. The Open field is a fast and relatively easy test that provides a variety of behavioral information ranging from general ambulatory ability to data regarding the emotionality of the subject animal. Thigmotaxis refers to a specific behavior of animals (i.e., to stay close to walls when exploring an open space). Such behavior can be assessed with the open field test, which is a well-established indicator of fear, an anxiety-related emotional behavior. Latency to center measures the animal's tendency to avoid the arena center. The latter measure is considered anxiety related, based on the assumption that the arena center is more threatening for rodents than its periphery. In this

study, *Fmr1* KO2 mice treated with Compound 1 (at 30 mg/kg) show a significant reversal to WT levels. Bars indicate mean values (mean ± SEM). Points correspond to values from individual mice. Asterisks represent significant change; ns, not significant; ****P < 0.0001. n = 15.

6.5.2. Sociability: three chamber partition test

The partition test is a measure of social recognition (SR) that works on the same principle as novel object recognition (NOR), except in this instance the test animals need to differentiate between a novel and a familiar mouse. The *Fmr1* KO2 vehicle-treated mice showed no preference for either the novel or the familiar mouse, as demonstrated by their low score. The 30 mg/kg Compound 1-treated group was able to reverse the SR deficit in *Fmr1* KO2 mice back to comparable levels observed for the WT vehicle group indicating improvement in social memory formation. The effects of single administration of vehicle or Compound 1 (30 mg/kg, i.p.) on sociability are presented in Figure 4. Asterisks represent significant change; ns, not significant; ****P < 0.0001. n = 15.

6.5.3. Memory and learning: novel object recognition test

Cognition was assessed using NOR, which tests an animal's ability to differentiate between a familiar object and a novel object. If the test animal can differentiate between the two objects, it will naturally show a preference for investigating the novel object. The *Fmr1* KO2 vehicle-treated mice cannot recall interacting with the familiar object, and as a result, the time spent investigating both objects are equivalent resulting in a low score. Compound 1 was able to reverse the cognitive deficit in *Fmr1* KO2 mice for the NOR task. The effects of single administration of vehicle or Compound 1 (30 mg/kg, i.p.) on memory and learning are presented in Figure 5 (NOR, exploration time) and Figure 6 (NOR, discrimination index). Asterisks represent significant change; ns, not significant; ****P < 0.0001. n = 15.

6.5.4. Anxiety: hyponeophagia

Mice and rats cannot vomit, due to the tightness of the cardiac sphincter of the stomach, so to overcome the problem of potential food toxicity they have evolved a strategy of first ingesting only very small amounts of novel substances. Hyponeophagia was used in this study as a way of measuring anxiety. A highly palatable but novel substance, such as sweetened condensed milk was offered to the mice in a novel situation [Deacon, Rob M J. (2011). Hyponeophagia: a measure of anxiety in the mouse. Journal of visualized experiments: JoVE, 51: 2613]. The latency to consume a defined amount of the new food is then measured.

The effects of single administration of vehicle or Compound 1 (30 mg/kg, i.p.) on anxiety are presented in Figure 7 (asterisks represent significant change; ns, not significant; ****P < 0.0001. n = 15). *Fmr1* KO2 mice treated with vehicle showed an increased latency to drink the condensed milk (anxious) when compared to WT littermate mice treated with vehicle. Compound 1 at 30 mg/kg significantly improves anxiety in *Fmr1* KO2 mice in the latency to drink the condensed milk (ns; p = 0.0753).

**6.6. Conclusions**

Single administration of Compound 1 at 30 mg/kg (i.p) in the *Fmr1* KO2 mice model of fragile X syndrome demonstrates a broad range of therapeutic effects. The compound displays broad efficacy across multiple cognition and behaviour measures, including the reduction of anxiety-like behaviour and hyperactivity, the improvement of sociability behaviour, and the attenuation of the dysregulation of recognition memory in the *Fmr1* KO2 mice model. The observation of clustering of mice in the open field test among treated *Fmr1* KO2 mice suggests that there may be a notable response to the treatment. The clustering behavior could suggest that the treatment is having a consistent effect on the treated *Fmr1* KO2 mice, leading to a more uniform response across individuals in the group.

Comparison between treated *Fmr1* KO2 and WT mice pointed out to the reversion of the cognition and behaviour pattern of *Fmr1* KO2 mouse phenotype to the WT phenotype. Thus, Compound 1 span across the brain as each of these assays target different brain regions and connections.

As conclusion, Compound 1 improved cognition and behaviour in a mouse model of FXS, both significantly affected in FXS patients.

**Example 7. Evaluation of the therapeutic activity in a mouse model of Rett Syndrome (RS)**

**7.1. Methods and experimental designs**

7.1.1. Animals

B6.129P2(C)-*Mecp2*$^{tm1.1Bird/J}$ *(Mecp2* KO) female mice were purchased from The Jackson Laboratory. These mice have been instrumental in understanding the cellular and molecular mechanisms underlying RS since they exhibit phenotypes similar to human conditions, such as abnormal brain morphology, reduced neuron size and density, and motor coordination issues. *Mecp2* KO mice were socially housed in numbers of two to five littermates.

Mice had access to food and water *ad libitum* in controlled laboratory conditions with temperature maintained at 22 ± 1°C and humidity at 55 ± 10% on a 12h light/dark cycle (lights off 20:00 h).

7.1.2. Cognition - Novel Object Recognition (NOR) test

The NOR test is a memory test that has been widely used to investigate the neurobiology of memory. The apparatus consisted of a rectangular open field arena (38 cm long × 38 cm wide × 38 cm high) made of black Plexiglas. Animals' behavior was monitored using System MotorActivity Record and Tracking software (SMART, Panlab HarvardApparatus).

On the initial day, the mice underwent a 5 min habituation period in the arena. The following day, they were introduced to two identical objects and allowed a 10 min exploration period. The duration of exploration for each object was recorded, and any subject not engaging in at least 10 seconds of exploration was excluded from further analysis. On the third day, during the test session, one of the familiar objects was replaced with a new one for a 5 min evaluation. The discrimination index was computed by subtracting the time spent exploring the familiar object from the time spent on the novel object, then dividing by the total exploration time. Exploration was only noted when the mouse's nose was within 2 cm of an object, directed towards it, and was manually quantified by the experimenter. Any behavior such as sitting or resting against the object did not count as exploration. All objects, made of plastic to ensure similar levels of exploration, and the arena were thoroughly cleaned with 70% ethanol between sessions to eliminate olfactory cues. The total exploration time served as an indicator of overall activity within the open field. These behavioral experiments were conducted during the light phase of the light/dark cycle by trained observers under blind conditions. All sessions were recorded for further analysis.

All mice were individually handled and habituated to the investigator during three days before the experiment.

Acute intraperitoneal injection of the vehicle or Compound 1 (30 mg/kg) was performed immediately after the familiarization session.

• *Compound administration*

Test compound was diluted in saline and administered intraperitoneally at a dose 30 mg/kg. Fresh solutions were prepared daily.

7.1.3. Behaviour - Social behavior assay

Social behavior test is designed to measure social interaction and preference for social novelty in mice. This test is particularly useful in assessing social behaviors that are indicative of conditions such as autism spectrum disorder in genetic mouse models. The apparatus consisted of an arena (70 cm long × 70 cm wide × 30 cm high made of Plexiglas. Animals' behavior was monitored using System Motor Activity Record and Tracking software (SMART, Panlab Harvard Apparatus).

Animals were habituated for 5 min to an arena containing two empty transparent cylinders made of Plexiglas (30 cm diameter × 40 cm high). The cylinders were perforated to allow interaction points and were located on two corners of the open field. After 24h, animals were placed in the same arena containing the two cylinders: one empty and the other one containing a stranger, a juvenile individual of the same genotype of the animal being tested. Exploration towards the stranger individual and empty cylinder was registered for 10 min. On day 3, animals were placed in the arena. In this case, one of the cylinders contained a new strange individual while the other contained the same animal as in day 2 (familiar one). Exploration time of both cylinders were quantified for 10 min. Exploratory behavior was defined as the animal directing its nose towards the cylinder at a distance of < 2 cm. Time spent at the proximal zone to the cylinder was also registered as an index of exploratory behavior. The discrimination index was calculated as time exploring the novel stimulus mouse - time exploring the familiar stimulus mouse / total time of exploration × 100. After every session, both cylinders and arena were cleaned with 70% ethanol to avoid olfactory cues. These behavioral experiments were conducted during the light phase of the light/dark cycle by trained observers under blind conditions. All sessions were recorded for further analysis. All mice were individually handled and habituated to the investigator during three days before the experiment.

Acute intraperitoneal injection of the vehicle or Compound 1 (30 mg/kg) was performed immediately after the sociability session.

• *Compound administration*

Test compound was diluted in saline and administered intraperitoneally at a dose 30 mg/kg. Fresh solutions were prepared daily.

### 7.2. Results and conclusions

7.2.1. Novel Object Recognition (NOR) test

On day 1, animals were habituated for 5 min to an open field arena. The following day, they were introduced to two identical objects and allowed a 10 min exploration period. On the third day, during the test session, one of the familiar objects was replaced with a new one for a 5 min evaluation. Acute intraperitoneal injection of the test articles was performed immediately after the sociability session.

Treated *Mecp2* KO mice showed a complete rescue of recognition memory, measured by the discrimination index, compared to *Mecp2* KO mice treated with vehicle (Figure 8). Remarkably, discrimination index in *Mecp2* KO mice treated with Compound 1 shown similar values to the WT mice. On the boxplots, the horizontal line indicates the median, the box indicates the first to third quartile of expression and whiskers indicate 1.5 × the interquartile range. Two-way ANOVA, Tukey HSD as *post hoc.* $^*P < 0.05$.

7.2.2. Social behavior assay

On day 1, animals were habituated for 5 min to an arena containing two empty transparent cylinders. After 24h, animals were placed in the same arena containing the two cylinders: one empty and the other one containing a stranger, a juvenile individual of the same genotype of the animal being tested, for 10 minutes. On day 3, animals were placed in the arena. In this case, one of the cylinders contained a new strange individual while the other contained the same animal as in day 2 (familiar one). Exploration time of both cylinders were quantified for 10 min. Acute intraperitoneal injection of test articles was performed immediately after the sociability session.

Treated *Mecp2* KO mice showed a significantly higher social recognition index as compared to *Mecp2* KO mice treated with vehicle (Figure 9). Remarkably, *Mecp2* KO mice treated with Compound 1 rescued social recognition to WT values. On the boxplots, the horizontal line indicates the median, the box indicates the first to third quartile of expression and whiskers indicate 1.5 × the interquartile range. Two-way ANOVA, Tukey HSD as *post hoc.* $^*P < 0.05$, $^{**} P < 0.01$, $^{***} P < 0.001$.

As conclusion, Compound 1 improved cognition and behaviour in a mouse model of RS, a relevant impaired feature in RS patients.

### Example 8. Pharmaceutical Compositions

The present invention also includes pharmaceutically active compositions comprising compounds of formula (I) and a physiological carrier. The active compositions can be administered either orally (solids or liquids), transdermal, subcutaneously, parenterally, locally (ointments, creams, gels, plasters, powders), as drops or as a nasal or buccal compositions.

The following examples of pharmaceutical compositions are offered with the understanding that they are in no way limiting of the invention.

### Formulation Example 1. Compositions for parenteral administration

These compositions may comprise physiologically acceptable sterile aqueous or non-aqueous solutions, emulsions, suspensions (in case of intramuscular or implants) and powders for reconstitution as injectable dosage forms.

The compounds of the invention in powder form may be diluted in phosphate buffered saline comprising polysorbate 80, potassium chloride, monobasic phosphate, sodium chloride, dibasic sodium phosphate and water for injection prepared to the desired proportion and volume.

Liposome injections for intrathecal use may be prepared in a sterile, injectable dispersion of compounds of formula (I), encapsulated in multi-vesicular lipid-based particles.

### Formulation Example 2. Tablets and capsules for oral administration

Tablets may be prepared containing as inactive ingredients lactose, magnesium stearate, hydroxypropylmethylcellulose, polyethylene glycol, povidone, sodium starch glycolate and titanium dioxide.

Capsules may be prepared containing as inactive ingredients talcum, sodium lauryl sulfate, colloidal silicon dioxide, magnesium stearate, titanium dioxide (E171), hard gelatin or hydroxypropylmethylcellulose capsule, black ink, propylene glycol and shellac.

**Formulation Example 3. Liquid compositions for oral administration**

Aqueous based oral solutions may be prepared containing as inactive ingredients citric acid, sodium citrate, benzoic acid, fruit flavorings, hydroxyethylcellulose, sorbitol, and water.

Oral suspensions may be prepared containing as inactive ingredients glycerin, carboxymethylcellulose sodium, hydroxyethylcellulose, citric acid, sodium citrate, benzoic acid, simethicone, polysorbate 80, flavorings, and water.

## Claims

1. A compound having formula (I)

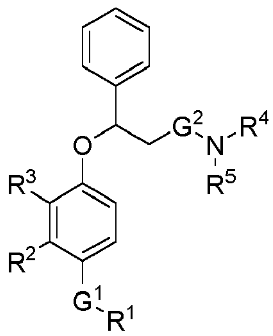

(I)

wherein,

- $R^1$ is selected from the group consisting of -OR and -NR'R", wherein R is selected from the group consisting of hydrogen, glycosyl, acyl and $C_1$-$C_6$ alkyl, and R' and R" are independently selected from the group consisting of hydrogen and $C_1$-$C_6$ alkyl,
- $R^2$ is selected from the group consisting of H and OH,
- $R^3$ is selected from the group consisting of H and OH,
- $R^4$ is selected from the group consisting of H, $-CH_3$ and OH,
- $R^5$ is selected from the group consisting of H and $-CH_3$,
- $G^1$ is a divalent linker comprising two carbon atoms and at least one oxygen atom selected from the group consisting of $-CH_2$-CH(OH)-, $-CH_2$-C(=O)-, -CH=C(OH)-, -CH(OH)-CH(OH)-, -CH(OH)-C(=O)-, and -C(=O)-C(=O)-, and
- $G^2$ is selected from the group consisting of $-CH_2$- and -C(=O)-,

or a pharmaceutically acceptable salt, hydrate or stereoisomer thereof.

2. A compound according to claim 1, wherein $G^1$ is selected from the group consisting of $-CH_2$-C(=O)-, -CH(OH)-C(=O)- and -C(=O)-C(=O)-, preferably $-CH_2$-C(=O)-.

3. A compound according to any one of claims 1 to 2, wherein at least one of $R^2$ and $R^3$ is H, preferably both $R^2$ and $R^3$ are H.

4. A compound according to any one of claims 1 to 3, wherein $R^4$ is H.

5. A compound according to any one of claims 1 to 4, wherein $R^5$ is $-CH_3$.

6. A compound according to any one of claims 1 to 5, wherein $G^2$ is $-CH_2$-.

**7.** A compound according to any one of claim 1 to 6, wherein $R^1$ is -OR, preferably -OH.

**8.** A compound according to claim 1, wherein $G^1$ is selected from the group consisting of $-CH_2-C(=O)-$ and-C(=O)-C(=O)-, $R^1$ is -OR, $R^2$, $R^3$ and $R^4$ are H, $R^5$ is $-CH_3$ and $G^2$ is $-CH_2-$.

**9.** A compound of formula (I) according to claim 1, which is selected from group consisting of:

- 2-(4-(3-(methylamino)-1-phenylpropoxy)phenyl)acetic acid
- 2-(4-(3-(methylamino)-1-phenylpropoxy)phenyl)ethane-1,1-diol
- 2-(4-(3-(methylamino)-1-phenylpropoxy)phenyl)ethene-1,1-diol
- 2-hydroxy-2-(4-(3-(methylamino)-1-phenylpropoxy)phenyl)acetic
- 2-(2-hydroxy-4-(3-(methylamino)-1-phenylpropoxy)phenyl)acetic acid
- 2-(3-hydroxy-4-(3-(methylamino)-1-phenylpropoxy)phenyl)acetic acid
- 2-(4-(3-(methylamino)-1-phenylpropoxy)phenyl)-2-oxoacetic acid
- 2-(4-(3-amino-1-phenylpropoxy)phenyl)ethane-1,1-diol
- 2-(4-(3-amino-1-phenylpropoxy)phenyl)acetic acid
- 2-hydroxy-1-(4-(3-(methylamino)-1-phenylpropoxy)phenyl)ethan-1-one
- methyl 2-(4-(3-(methylamino)-1-phenylpropoxy)phenyl)acetate
- 2-(4-(3-(methylamino)-1-phenylpropoxy)phenyl)acetamide

or a pharmaceutically acceptable salt, hydrate or stereoisomer thereof.

**10.** A pharmaceutical composition comprising a compound as defined in anyone of claims 1 to 9 and one or more pharmaceutically acceptable carriers.

**11.** A compound as defined in anyone of claims 1 to 9 for use as a medicament.

**12.** A compound as defined in any one of claims 1 to 9 for use in the treatment of nervous system disorders associated with cognitive deficits and behavioural dysfunctions.

**13.** A compound for use according to claim 12, wherein the nervous system disorder associated with cognitive deficits and behavioural dysfunctions is selected from the group consisting of fragile X syndrome, Rett syndrome, Down syndrome, DiGeorge syndrome (22q11.2 deletion syndrome), Prader-Willi syndrome, Angelman syndrome, autism spectrum disorder, Asperger syndrome, attention deficit hyperactivity disorder, anxiety, depression, schizophrenia, Alzheimer's disease, Huntington's disease, Parkinson's disease.

**14.** A compound for use according to claim 13, wherein the nervous system disorder associated with cognitive deficits and behavioural dysfunctions is fragile X syndrome or Rett syndrome, preferably fragile X syndrome.

**15.** A combination comprising a compound as defined in anyone of claims 1 to 9 and another drug selected from the group consisting of drugs for the treatment of nervous system disorders or their clinical manifestations: anxiolytics and antidepressants, such as sertraline, fluoxetine, citalopram, escitalopram, trazodone, bupropion, cannabidiol, paroxetine, duloxetine, buspirone, venlafaxine, desvenlafaxine, olanzapine, mirtazapine, psilocybin and atomoxetine; antipsychotics, such as aripiprazole, risperidone, quetiapine and olanzapine; tranquilizers, such as clonazepam and lorazepam; hypnotics, such as zolpidem and melatonin; stimulants, such as methylphenidate, amphetamine, amphetamine salts and L-acetylcarnitine; non-stimulants, such as clonidine and guanfacine; cognition enhancers, such as donepezil, zatolmilast, trofinetide and metformin; antiepileptics and anticonvulsants, such as levetiracetam, oxcarbazepine, valproic acid, valproate, carbamazepine, lamotrigine and topiramate; and 2-[4-[3-(methylamino)-1-phenylpropoxy]phenyl]ethanol or a pharmaceutically acceptable salt, hydrate or stereoisomer thereof.

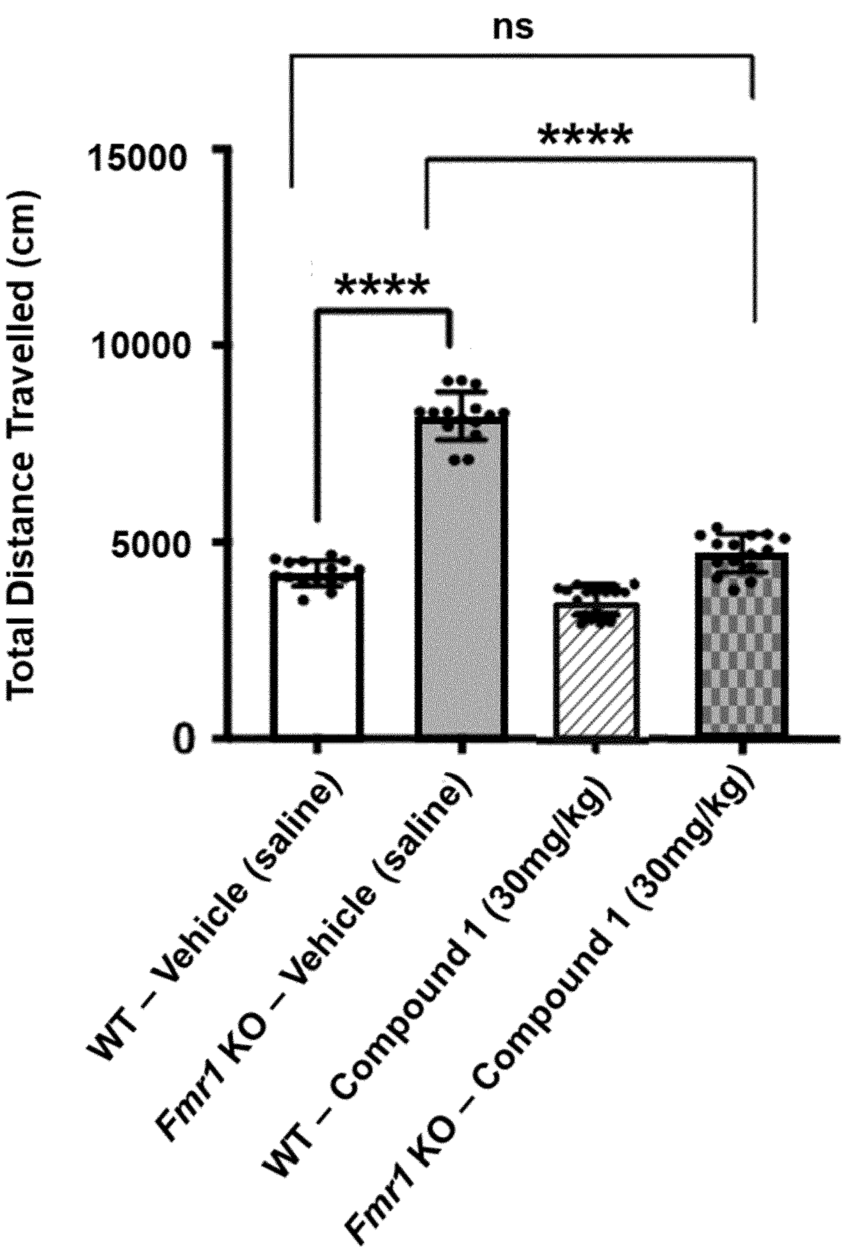

ns
****
****
Total Distance Travelled (cm)
15000
10000
5000
0
WT – Vehicle (saline)
Fmr1 KO – Vehicle (saline)
WT – Compound 1 (30mg/kg)
Fmr1 KO – Compound 1 (30mg/kg)


FIG. 1

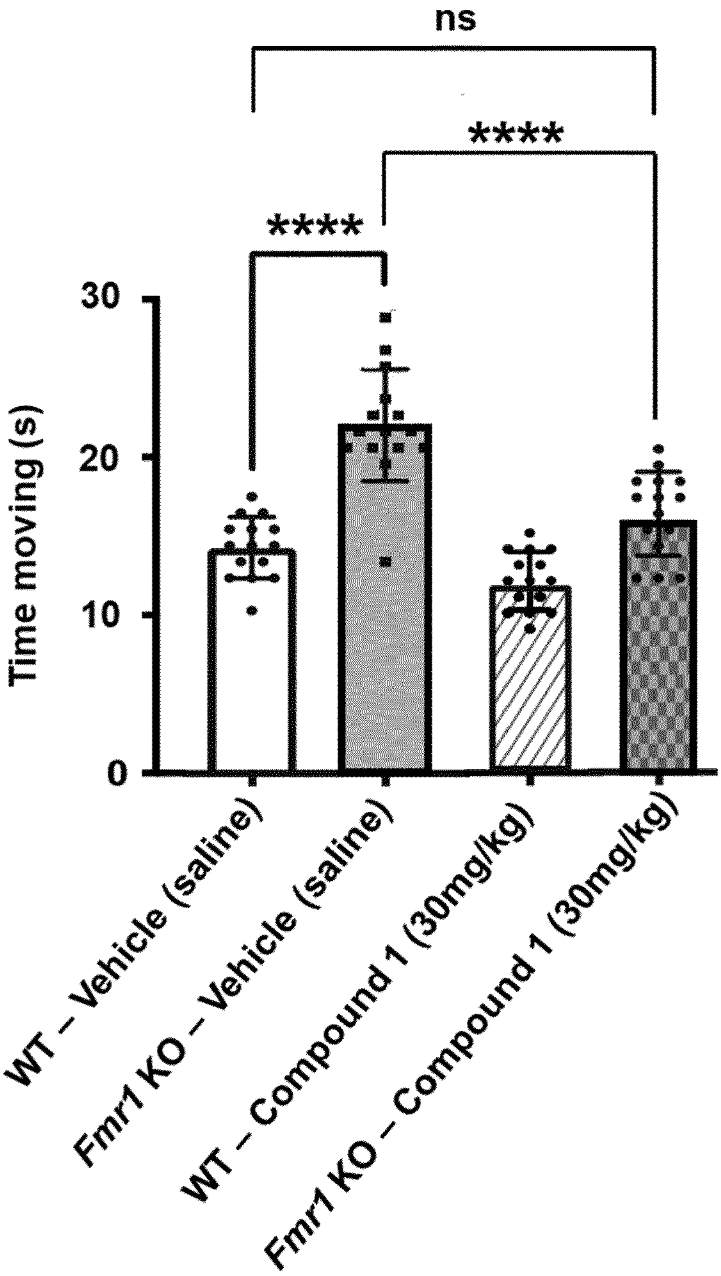
ns
****
****
Time moving (s)
30
20
10
0
WT – Vehicle (saline)
Fmr1 KO – Vehicle (saline)
WT – Compound 1 (30mg/kg)
Fmr1 KO – Compound 1 (30mg/kg)

FIG. 2

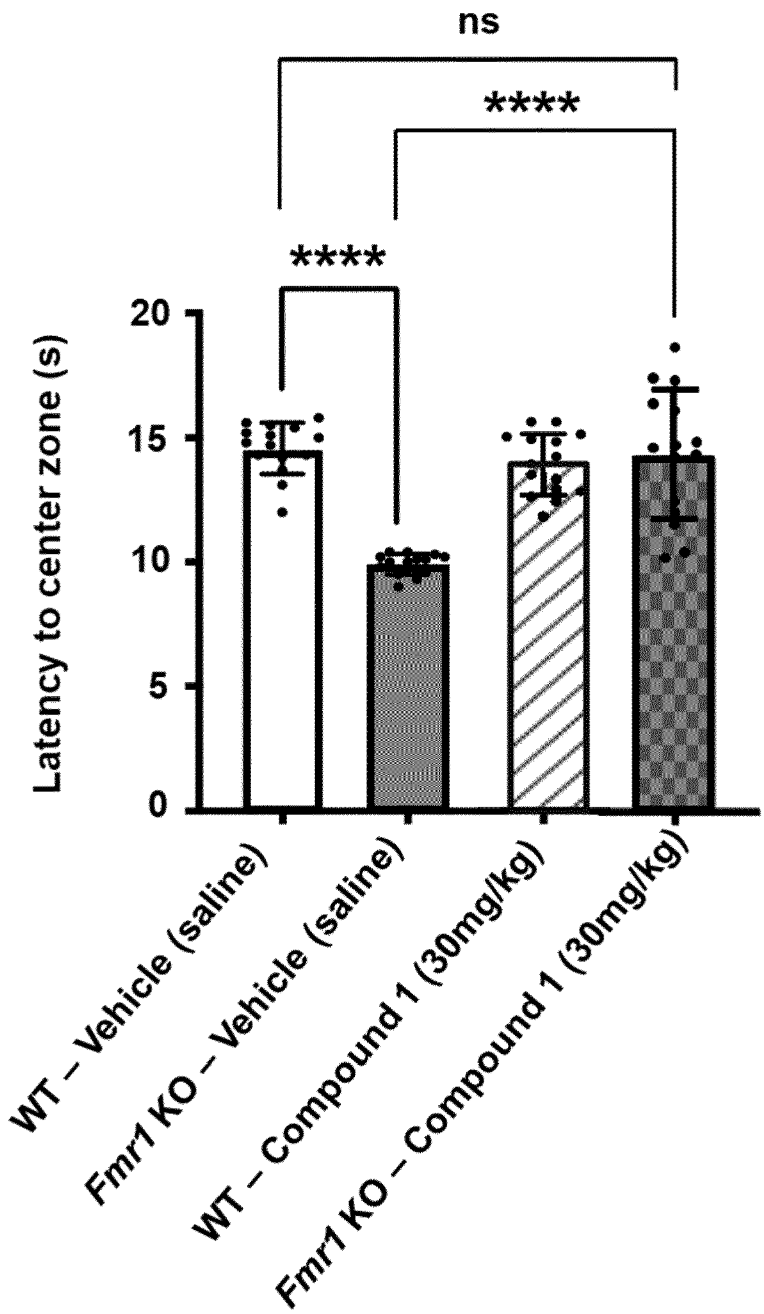
ns
****
****
Latency to center zone (s)
20
15
10
5
0
WT – Vehicle (saline)
Fmr1 KO – Vehicle (saline)
WT – Compound 1 (30mg/kg)
Fmr1 KO – Compound 1 (30mg/kg)

FIG. 3

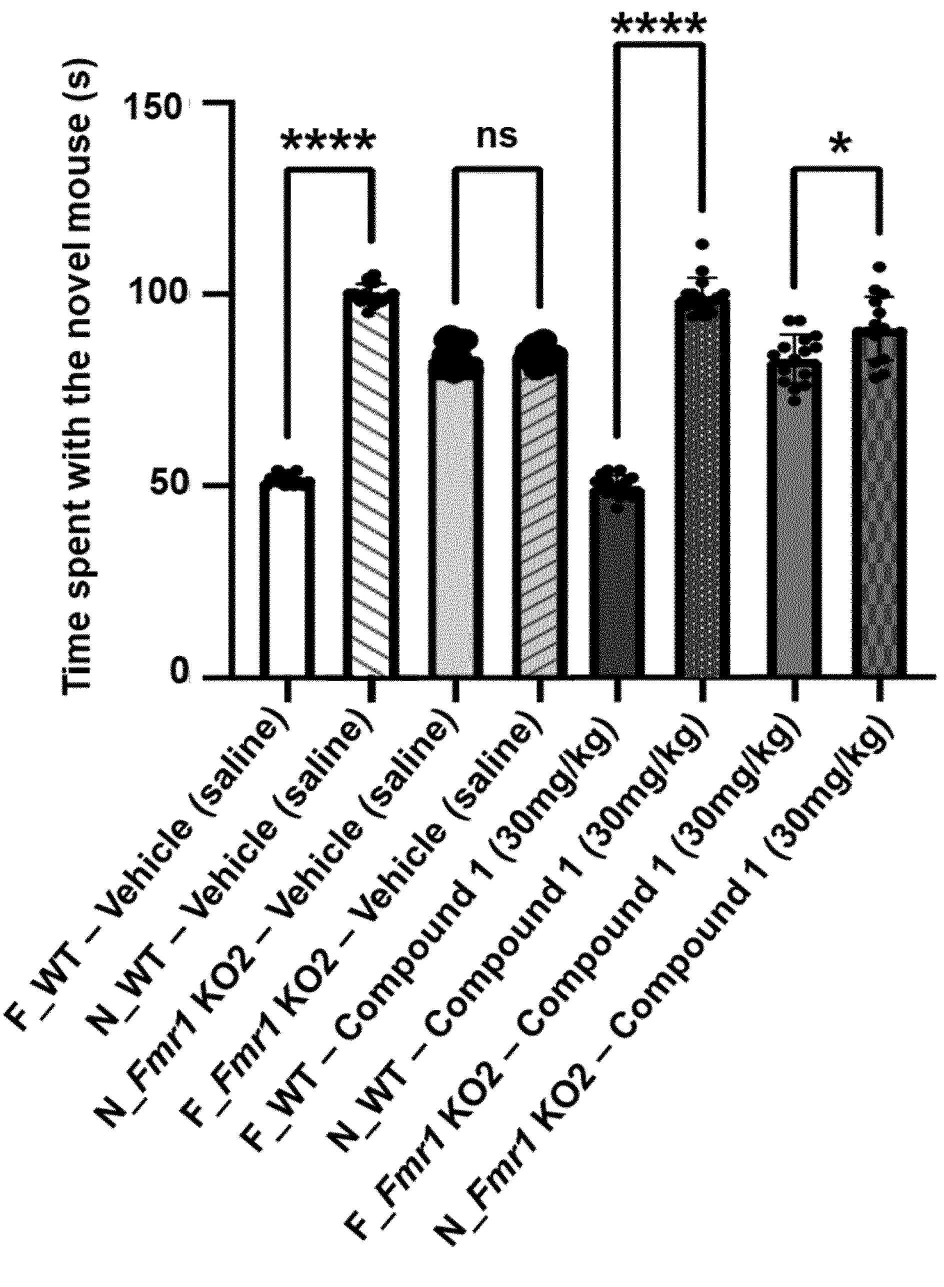
Time spent with the novel mouse (s)
150
100
50
0
****
ns
****
*
F_WT – Vehicle (saline)
N_WT – Vehicle (saline)
N_Fmr1 KO2 – Vehicle (saline)
F_Fmr1 KO2 – Vehicle (saline)
F_WT – Compound 1 (30mg/kg)
N_WT – Compound 1 (30mg/kg)
F_Fmr1 KO2 – Compound 1 (30mg/kg)
N_Fmr1 KO2 – Compound 1 (30mg/kg)

FIG. 4

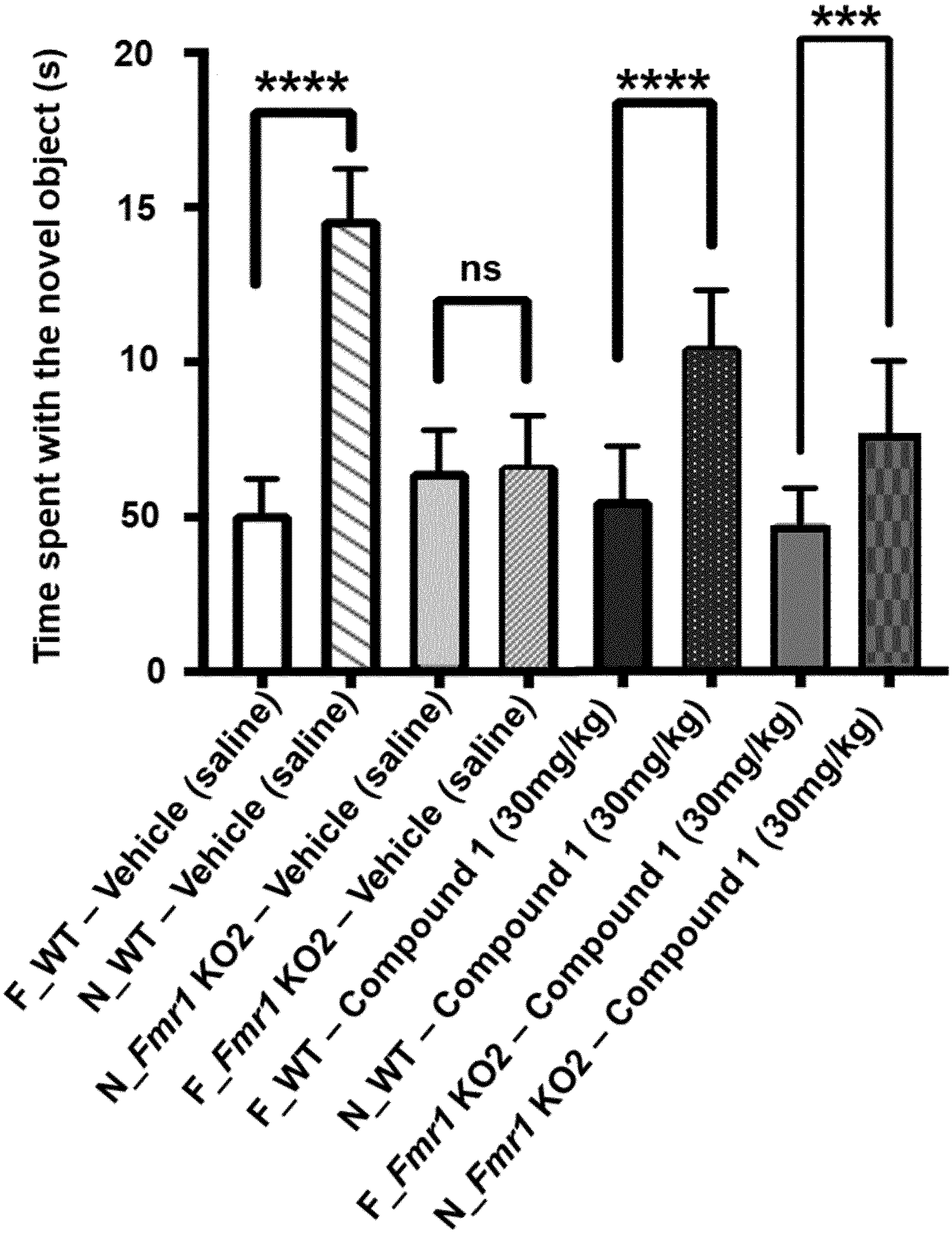
Time spent with the novel object (s)
20
15
10
50
0
****
ns
****
***
F_WT – Vehicle (saline)
N_WT – Vehicle (saline)
N_Fmr1 KO2 – Vehicle (saline)
F_Fmr1 KO2 – Vehicle (saline)
F_WT – Compound 1 (30mg/kg)
N_WT – Compound 1 (30mg/kg)
F_Fmr1 KO2 – Compound 1 (30mg/kg)
N_Fmr1 KO2 – Compound 1 (30mg/kg)

FIG. 5

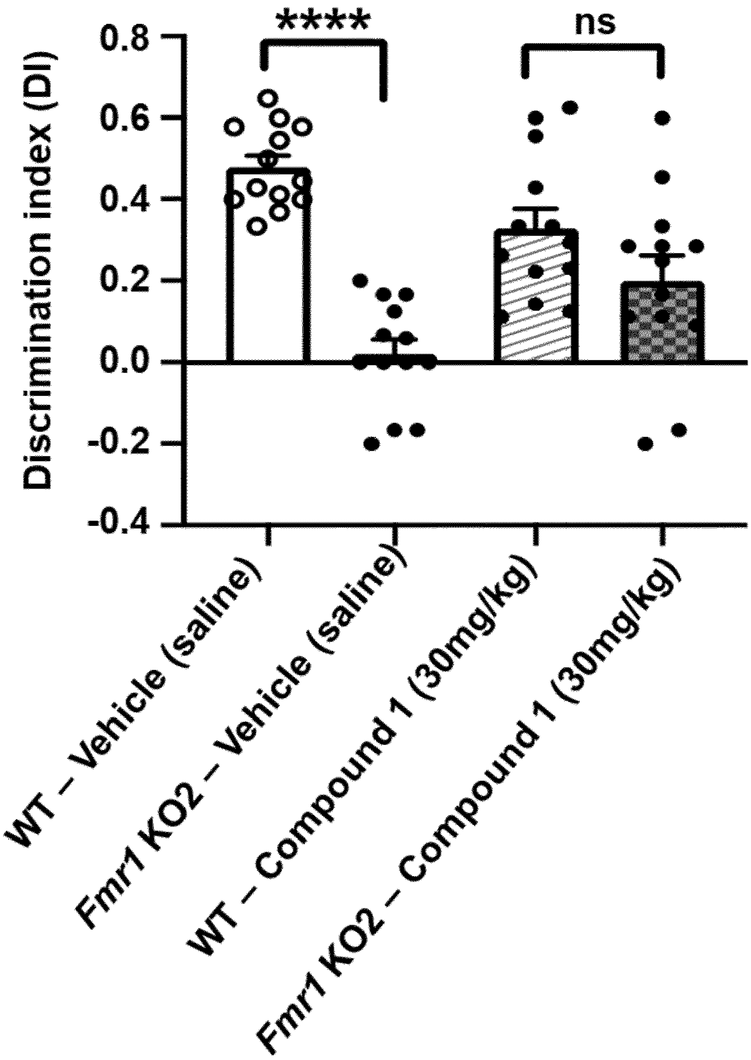
****
ns
0.8
0.6
0.4
0.2
0.0
-0.2
-0.4
Discrimination index (DI)
WT – Vehicle (saline)
Fmr1 KO2 – Vehicle (saline)
WT – Compound 1 (30mg/kg)
Fmr1 KO2 – Compound 1 (30mg/kg)

FIG. 6

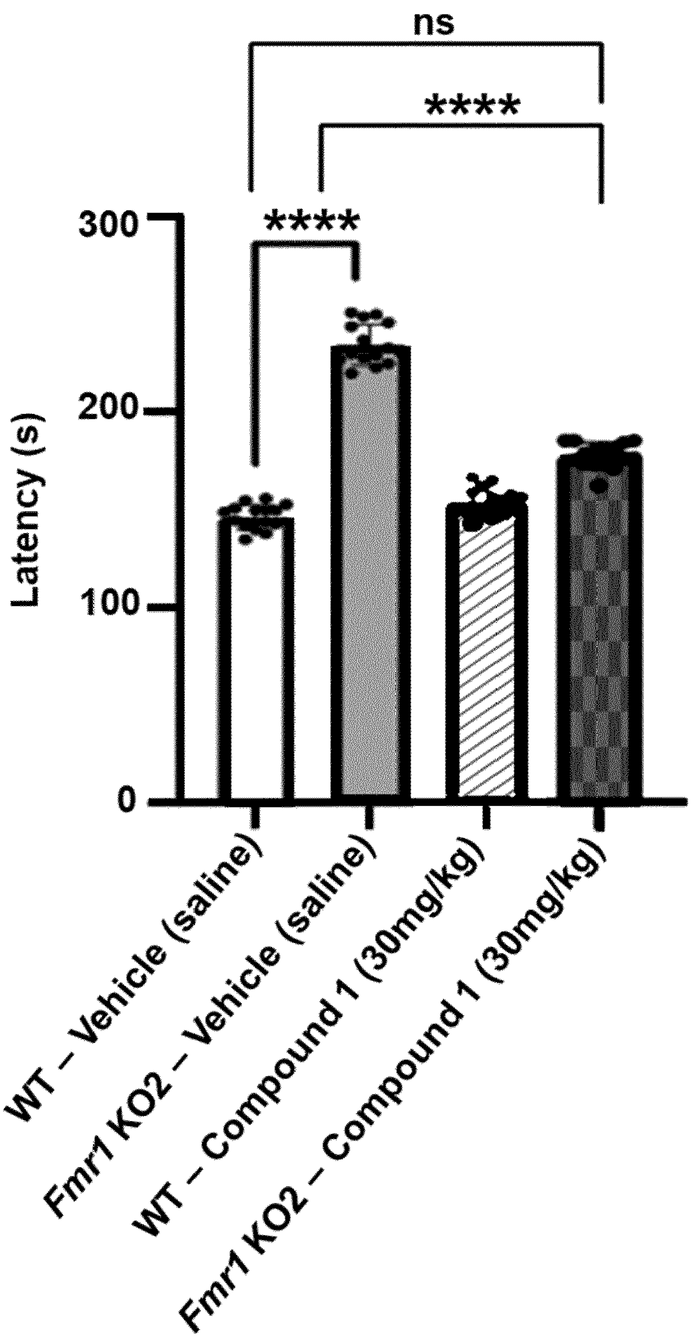
ns
****
****
300
200
100
0
Latency (s)
WT – Vehicle (saline)
Fmr1 KO2 – Vehicle (saline)
WT – Compound 1 (30mg/kg)
Fmr1 KO2 – Compound 1 (30mg/kg)

FIG. 7

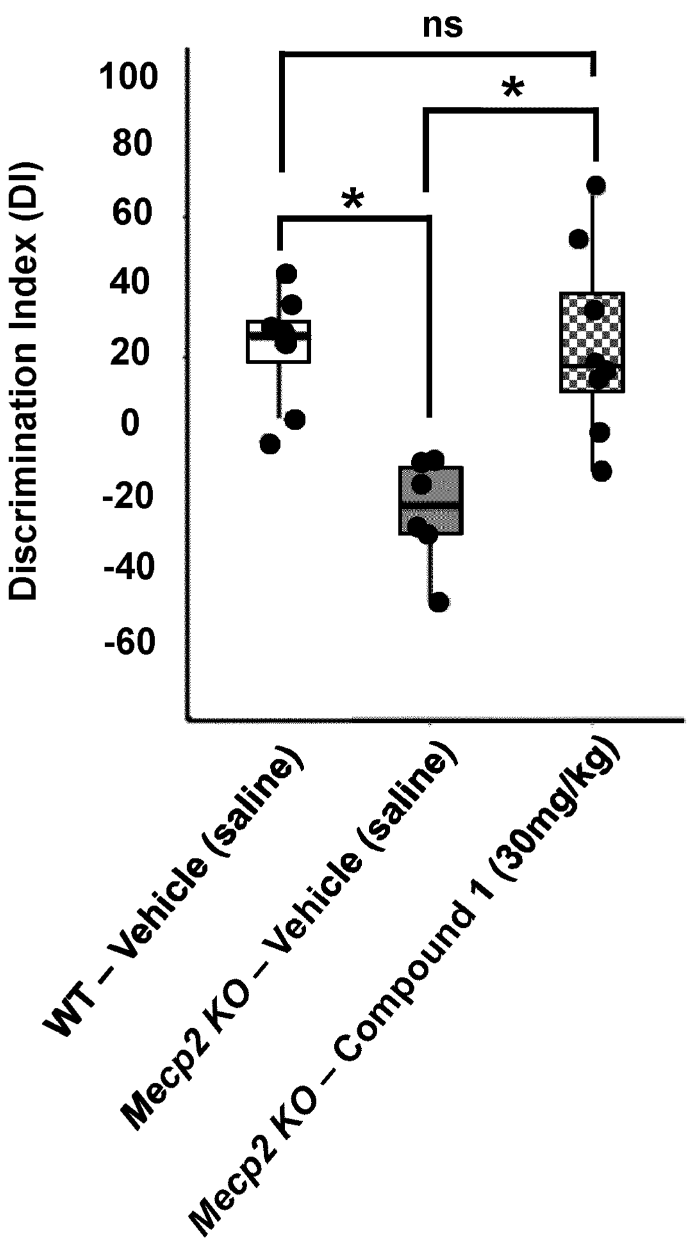
ns
*
*
Discrimination Index (DI)
100
80
60
40
20
0
-20
-40
-60
WT – Vehicle (saline)
Mecp2 KO – Vehicle (saline)
Mecp2 KO – Compound 1 (30mg/kg)

FIG. 8

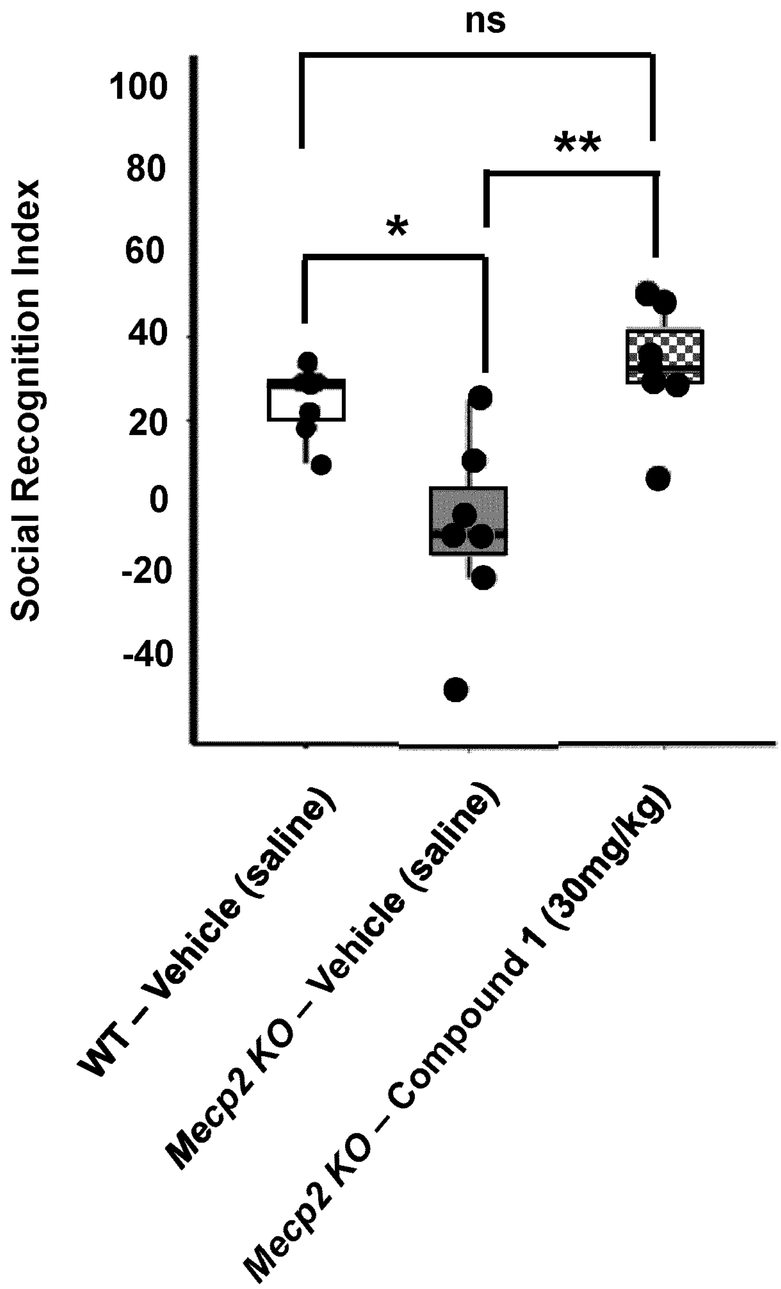
ns
**
*
100
80
60
40
20
0
-20
-40
Social Recognition Index
WT – Vehicle (saline)
Mecp2 KO – Vehicle (saline)
Mecp2 KO – Compound 1 (30mg/kg)

FIG. 9

Europäisches Patentamt
European Patent Office
Office européen des brevets

# EUROPEAN SEARCH REPORT

Application Number
EP 24 38 2738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2015/344408 A1 (PROUS JOSEP R [ES] ET AL) 3 December 2015 (2015-12-03)<br>* formula (I) *<br>* claims 35-38, 41-42, 47 *<br>* paragraphs [0267], [0421] *<br>* page 28 *<br>* table 4 *<br>& WO 2014/096377 A1 (PROUS INST FOR BIOMEDICAL RES S A [ES])<br>26 June 2014 (2014-06-26)<br>----- | 1-15 | INV.<br>C07C217/48<br>A61P25/00 |
| A | WO 2020/259787 A1 (UNIV COPENHAGEN [DK])<br>30 December 2020 (2020-12-30)<br>* formula IV *<br>* claims 1, 24-26 *<br>----- | 1-15 | |
| | | | TECHNICAL FIELDS SEARCHED (IPC) |
| | | | C07C<br>A61P |

The present search report has been drawn up for all claims

1

| Place of search | Date of completion of the search | Examiner |
|---|---|---|
| The Hague | 31 January 2025 | Kövecs, Monika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 38 2738

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2025

| Patent document cited in search report | Publication date | Patent family member(s) | Publication date |
|---|---|---|---|
| US 2015344408 A1 | 03-12-2015 | AU 2013366502 A1 | 09-07-2015 |
| | | CA 2895592 A1 | 26-06-2014 |
| | | DK 2945702 T3 | 18-04-2017 |
| | | EP 2745876 A1 | 25-06-2014 |
| | | EP 2945702 A1 | 25-11-2015 |
| | | ES 2621960 T3 | 05-07-2017 |
| | | HR P20170539 T1 | 16-06-2017 |
| | | HU E032461 T2 | 28-09-2017 |
| | | JP 6302929 B2 | 28-03-2018 |
| | | JP 2016504992 A | 18-02-2016 |
| | | PT 2945702 T | 20-04-2017 |
| | | US 2015344408 A1 | 03-12-2015 |
| | | US 2017239197 A1 | 24-08-2017 |
| | | WO 2014096377 A1 | 26-06-2014 |
| WO 2020259787 A1 | 30-12-2020 | AU 2020306249 A1 | 17-02-2022 |
| | | CA 3140704 A1 | 30-12-2020 |
| | | CN 114008013 A | 01-02-2022 |
| | | EP 3989957 A1 | 04-05-2022 |
| | | IL 289012 A | 01-02-2022 |
| | | JP 2022538569 A | 05-09-2022 |
| | | US 2022339131 A1 | 27-10-2022 |
| | | WO 2020259787 A1 | 30-12-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014096377 A1

### Non-patent literature cited in the description


- Cognitive Deficits. **DHAKAL A** ; **BOBRIN BD**. StatPearls. StatPearls Publishing, 14 February 2023
- **DIONNE O** ; **CORBIN F.** An "omic" overview of fragile X syndrome. *Biology (Basel)*, 2021, vol. 10 (5), 433
- **PROTIC DD** ; **AISHWORIYA R** ; **SALCEDO-ARELLANO MJ et al.** Fragile X syndrome: From molecular aspect to clinical treatment.. *Int J Mol Sci*, 2022, vol. 23 (4), 1935
- **DIONNE O** ; **CORBIN F**. An "omic" overview of fragile X syndrome. *Biology (Basel)*, 2021, vol. 10 (5), 433
- **BAGNI C** ; **ZUKIN RS**. A Synaptic Perspective of Fragile X Syndrome and Autism Spectrum Disorders. *Neuron*, 20 March 2019, vol. 101 (6), 1070-1088
- **LOPES, A. G.** ; **LOGANATHAN, S. K.** ; **CALIAPERUMAL, J.** Rett Syndrome and the Role of MECP2: Signaling to Clinical Trials. *Brain Sciences*, 2024, vol. 14 (2), 120
- **GOMES, A. R.** ; **FERNANDES, T. G.** ; **CABRAL, J. M.** ; **DIOGO, M. M.** Modeling Rett syndrome with human pluripotent stem cells: Mechanistic outcomes and future clinical perspectives. *International journal of molecular sciences*, 2021, vol. 22 (7), 3751
- **DIERSSEN M.** Down syndrome: the brain in trisomic mode. *Nat Rev Neurosci*, December 2012, vol. 13 (12), 844-58
- **BULL, M. J.** Down syndrome. *New England Journal of Medicine*, 2020, vol. 382 (24), 2344-2352
- **SMERCONISH S** ; **SCHMITT JE.** Neuroanatomical Correlates of Cognitive Dysfunction in 22q11.2 Deletion Syndrome. *Genes (Basel)*, 2024, vol. 15 (4), 440
- **ANGULO MA** ; **BUTLER MG** ; **CATALETTO ME.** Prader-Willi syndrome: a review of clinical, genetic, and endocrine findings. *J Endocrinol Invest*, December 2015, vol. 38 (12), 1249-63
- **MARANGA C** ; **FERNANDES TG** ; **BEKMAN E** ; **DA ROCHA ST.** Angelman syndrome: a journey through the brain. *FEBS J.*, 2020, vol. 287 (11), 2154-2175
- **MARGOLIS SS** ; **SELL GL** ; **ZBINDEN MA** ; **BIRD LM.** Angelman Syndrome. *Neurotherapeutics*, 2015, vol. 12 (3), 641-50
- **LORD C** ; **BRUGHA TS** ; **CHARMAN T** ; **CUSACK J** ; **DUMAS G** ; **FRAZIER T** ; **JONES EJH** ; **JONES RM** ; **PICKLES A** ; **STATE MW**. Autism spectrum disorder. *Nat Rev Dis Primers*, 2020, vol. 6 (1), 5
- **HOSSEINI SA** ; **MOLLA M.** Asperger Syndrome.. StatPearls Publishing, 12 February 2024
- **MAGNUS W** ; **NAZIR S** ; **ANILKUMAR AC et al.** Attention Deficit Hyperactivity Disorder.. StatPearls Publishing, 08 August 2023
- **CHAND SP** ; **MARWAHA R.** Anxiety.. StatPearls Publishing, 24 April 2023
- **CHAND SP** ; **ARIF H.** Depression. StatPearls Publishing, 17 July 2023
- **PATEL KR** ; **CHERIAN J** ; **GOHIL K** ; **ATKINSON D.** Schizophrenia: overview and treatment options. *Pharmacy and Therapeutics*, 2014, vol. 39 (9), 638-45
- **MCCUTCHEON RA** ; **REIS MARQUES T** ; **HOWES OD.** Schizophrenia-An Overview. *JAMA Psychiatry*, 2020, vol. 77 (2), 201-210
- **BREIJYEH Z** ; **KARAMAN R.** Comprehensive Review on Alzheimer's Disease: Causes and Treatment. *Molecules*, 08 December 2020, vol. 25 (24), 5789
- **AJITKUMAR A** ; **DE JESUS O.** Huntington Disease.. StatPearls Publishing, 23 August 2023
- **ARMSTRONG MJ** ; **OKUN MS.** Diagnosis and Treatment of Parkinson Disease: A Review. *JAMA*, 2020, vol. 323 (6), 548-560
- **GREENE, T.W.** ; **WUTS, P.G.M.** Protecting Groups in Organic Synthesis. J. Wiley & Sons, 1999
- **PACHOLCZYK, T.** *Nature*, 1991, vol. 350, 350-354
- **PRISTUPA, Z.B. et al.** *Mol. Pharmacol.*, 1994, vol. 45, 125-135
- **TATSUMI, M. et al.** *Eur. J. Pharmacol.*, 1999, vol. 368, 277-283
- **PEROVIC, S.** ; **MULLER, W.E.G.** Arzneim-Forsch.. *Drug Res.*, 1995, vol. 45, 1145-1148
- **VERRICO C. et al.** *Psychopharmacology (Berl)*, 2005

- **MIENTJES E. J.** ; **NIEUWENHUIZEN I.** ; **KIRKPATRICK L.** ; **ZU T.** ; **HOOGEVEEN-WESTERVELD M.** ; **SEVERIJNEN L. et al.** The generation of a conditional Fmr1 knock out mouse model to study Fmrp function in vivo. *Neurobiol. Dis.*, 2006, vol. 21, 549-555
- **GAUDISSARD J** ; **GINGER M** ; **PREMOLI M** ; **MEMO M** ; **FRICK A** ; **PIETROPAOLO S.** Behavioral abnormalities in the Fmr1-KO2 mouse model of fragile X syndrome: The relevance of early life phases. *Autism Res.*, 2017, vol. 10 (10), 1584-1596
- **OLMOS-SERRANO J. L.** ; **CORBIN J. G.** ; **BURNS M. P.** The GABAA receptor agonist THIP ameliorates specific behavioral deficits in the mouse model of fragile X syndrome. *Dev. Neurosci.*, 2011, vol. 33, 395-403
- **DEACON, ROB M J.** Hyponeophagia: a measure of anxiety in the mouse. *Journal of visualized experiments: JoVE*, 2011, vol. 51, 2613